(19) Europäisches Patentamt
European Patent Office
Office européen des brevets

(11) EP 1 713 400 B1

(12) **EUROPEAN PATENT SPECIFICATION**

(45) Date of publication and mention of the grant of the patent:
**26.12.2012 Bulletin 2012/52**

(21) Application number: **05722446.1**

(22) Date of filing: **14.01.2005**

(51) Int Cl.:
***A61B 8/12*** *(2006.01)* ***A61M 5/00*** *(2006.01)*
***A61M 25/10*** *(2006.01)*

(86) International application number:
**PCT/US2005/001436**

(87) International publication number:
**WO 2005/070299 (04.08.2005 Gazette 2005/31)**

(54) **APPARATUS FOR MEDICAL IMAGING**

GERÄT FÜR DIE MEDIZINISCHE BILDDARSTELLUNG

APPAREIL POUR UNE IMAGERIE MEDICALE

(84) Designated Contracting States:
**AT BE BG CH CY CZ DE DK EE ES FI FR GB GR HU IE IS IT LI LT LU MC NL PL PT RO SE SI SK TR**

(30) Priority: **16.01.2004 US 536807 P**

(43) Date of publication of application:
**25.10.2006 Bulletin 2006/43**

(73) Proprietor: **Boston Scientific Scimed, Inc.**
**Maple Grove, MN 55311 (US)**

(72) Inventors:
- **NAGHAVI, Morteza**
**Houston, TX 77005 (US)**
- **KAKADIARIS, Ioannis, A.**
**Bellaire, TX 77401 (US)**

(74) Representative: **Golkowsky, Stefan**
**Pfenning, Meinig & Partner GbR**
**Patent- und Rechtsanwälte**
**Joachimstaler Strasse 10-12**
**10719 Berlin (DE)**

(56) References cited:
**US-A1- 2002 065 487** **US-A1- 2003 028 114**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## RELATED APPLICATIONS

## STATEMENT REGARDING GOVERNMENTAL RIGHTS

This invention was made in part with government support under Cooperative Agreement awarded by The National Science Foundation and the government may have certain rights in the subject matter disclosed herein.

## BACKGROUND OF THE INVENTION

### 1. Field of the Invention

The present invention relates to a system for detecting and localizing vasa vasorum or other microvessels associated with arteries, veins, tissues, organs and cancers in animals including humans.

More particularly, the present invention relates to a system including the steps of acquiring contrast-enhanced data and analyzing the acquired data to prepare a view of the anatomy and/or morphology a portion of an artery, vein, tissue, organ and/or cancer within the scope of the acquired data. In addition, the present disclosure relates to a mathematical method to enhance visualization of perfused vasa vasorum, other microvessels or other structures over time derived from the acquired data. In addition, the present disclosure relates to a mathematical method to numerically quantify aspects of the anatomy and/or morphology vessel interior a portion of an artery, vein, tissue, organ and/or cancer within the scope of the acquired data as a function of acquisition time. In addition, the present disclosure relates to a method for determining the extent of arterial blockage in coronary arteries or other arteries using contrast agents such as micro-bubble ultrasound contrast agents, acquiring an image sequence of the arteries before, during and after contrast agent profusion and analyzing the images to determining an extent of arterial blockage.

### 2. Description of the Related Art

Vulnerable plaques are subsets of atherosclerotic lesions that can rupture and create blood clots resulting in acute coronary syndrome, sudden cardiac death and/or stroke. Plaque inflammation plays a central role in plaque vulnerability to future complications (e.g., rupture, erosion, hemorrhage, distal emboli, and rapid progression). Finding a technology capable of imaging both plaque anatomy and/or morphology and activity (inflammation) is currently a major effort in the cardiology community.

Despite major advancements in the development of intravascular imaging techniques such as thermography, optical coherent tomography, near infrared spectroscopy, and magnetic resonance imaging, intravascular ultrasound (IVUS) remains the most useful technology widely-available to interventional cardiologists. IVUS is capable of providing more information related to multiple attributes of plaque than any other technology. However, one major drawback in IVUS imaging is that it lacks the ability to provide information about plaque inflammation.

The vasa vasorum are microvessels that nourish vessel walls. In conditions with extensive neovessel formations such as atherosclerotic plaques, tumor angiogenesis, and diabetic retinopathy, most are fragile and prone to leakage or rupture. There is abundant evidence indicating that angiogenesis is a pathophysiological response to injury. It has been known for decades that the degree of neovessel formation in any tissue is closely related to its metabolic activity as well as cell density and proliferation.

In the field of atherosclerosis, recent evidence suggests that proliferation of the vasa vasorum is a preceding or concomitant factor associated with plaque inflammation and instability. New concepts highlight the significance of leaking vasa vasorum and vasa vasorum-mediated intraplaque hemorrhage in plaque instability. According to this hypothesis, the lipid core inside the plaque results from the accumulation of cholesterol-rich membranes of red blood cells leaked out from vasa vasorum. Therefore, the fat buildup is likely to originate from the outside of the wall through vasa vasorum instead of from the inside (through diffusion from the lumen).

This phenomenon often enters into a vicious cycle and leads to plaque complications, e.g., rupture, erosion, hemorrhage, and stenosis. Although the role of red blood cell (RBC) cholesterol in atherosclerotic plaque formation was first noticed decades ago, new studies highlight the importance of vasa vasorum in plaque inflammation and vulnerability.

Another new concept was also introduced to study plaque inflammation beyond the intima and media layers. These studies show inflammation in the peri-adventitia fat area, particularly around the vasa vasorum, that was not recognized before. CT and MRI techniques have been used to image these processes, but to date none have been truly successful.

## Microbubbles

Recently, microcapillaries have been imaged using microbubble contrast enhanced ultrasound in the fields of cancer (tumor perfusion) and cardiovascular (myocardial perfusion). There has been a tremendous amount of research done on the clinical applications of ultrasonic contrast agents. Several forms of gas-filled microbubbles have been developed. In addition to non-targeted use of microbubbles for imaging bloodperfusion in microcapillaries, use of targeted microbubbles with specific ligands against markers ofdisease in various tissues allows molecular and cellular ultrasound imaging of the disease. These microbubble techniques are finding use in both non-invasive and invasive microbubble-targeted ultrasound imaging.

In previous intravascular ultrasound (IVUS) acquisition systems, images of the vasa vasorum consisted of video recordings of the cross-sectional interior of a vessel of interest inside an animal including a human subject at typical rates of 15 to 30 frames per second. The previous digitization method converted the resulting IVUS video to a sequence of 8-bit (255 gray-level) images utilizing the industry-standard Digital Imagine and Communications in Medicine (DICOM) file format. The ultrasound contrast agent used was Optison®, an agent composed of albumin microspheres filled with octafluoropropane gas. Cardiac-motion compensation was performed using techniques used in previous IVUS studies.

Thus, there is a need in the art for determining tissue site anatomy, morphology and/or functional features such as anatomy, morphology and/or functional features of coronary arteries having vulnerable atherosclerotic plaques using a detection system in which data is collected both prior to and after a data contrast event occur.

## SUMMARY OF THE INVENTION

The present invention in defined by a catheter apparatus comprising the features of claim 1. Preferred embodiments of this apparatus are represented in the dependent claims.

A system for medical imaging can include a guide catheter, and a micro-catheter having a contrast agent delivery subsystem and a power supply for the contrast agent delivery subsystem. The guide-catheter and micro-catheter are designed to be inserted into a vessel that either feeds blood to or removes blood from a body structure to be analyzed. The contrast agent delivery subsystem is adapted to introduce or inject a contrast agent into the vessel so that the agent profuses into the body structure to be analyzed. A probe is then positioned adjacent the body part and a first sequence of data of the body structure to be analyzed is acquired and stored. After the first data sequence is acquired, the contrast agent delivery subsystem is activated and a desired amount of the contrast agent is introduced into the vessel. Optionally and preferably, a second data sequence is acquired during the contrast agent delivery. After contrast delivery, a third data sequence is acquired. These three data sequences are then analyzed to determine morphological, anatomical and functional properties of the body part to be analyzed. The system can be used in different configurations. In one preferred configuration, the probe is associated with the micro-catheter and the data being acquired is associated with the structure surrounding the probe which is either an artery and its surrounding tissue or a vein and its surrounding tissue. Thus, the entire system is associated with the catheter apparatus. In another preferred configuration, the probe is not associated with the catheter apparatus used to inject the contrast agent, but is positioned in or adjacent a body structure that is fed by the vessel into which the contrast agent is introduced. This latter configuration can be practiced in two ways. First, the probe can be within a body structure near the site of contrast agent injection. Second, the probe can be external to a body structure near the site of contrast agent injection. For example, the catheter assembly can be inserted into a vein such as the coronary sinus, while the probe is positioned into an artery that includes vasa vasorum fed by the vein- an internal configuration. An example of an external configuration is the insertion of the contrast catheter assembly into an artery feeding a tissue site of interest and the probe is positioned on an exterior surface of the tissue site such as an intestinal lining, a site inside a mouth or a site on an esophagus, a site of a urinary tract, a site of a reproductive tract, a skin site or other external organ site or a cancer, tumor or cyst site.

A system for ultrasound imaging of a vessel can include a guide catheter, a micro-catheter having an ultrasound probe at or near its distal end, a microbubble delivery unit, a power supply for the ultrasound probe and an analyzer for extracting vessel morphological, anatomical and function data from a sequence of ultrasound images taken using the ultrasound probe, where the microbubble delivery unit includes a conduit having proximal end and a distal end with the proximal end connected to a microbubble supply having a controllable pump and with its distal end terminating in either the distal end of the guide-catheter probe or at or near a distal end of the micro-catheter.

## BRIEF DESCRIPTION OF THE DRAWINGS

The invention can be better understood with reference to the following detailed description together with the appended illustrative drawings in which like elements are numbered the same:

**Figures 1A-F** depict pictorially a method for imaging coronary arteries of this invention, where a guide-catheter and a micro-catheter having a magnetic imaging probe is positioned in a coronary artery and images taken before and

after contrast agent injection;

**Figures 2A-C** illustrates the different types of vasa vasorum;

**Figures 3** depicts pictorially a anatomy or morphology of a diseased coronary artery showing different types of plaques;

**Figures 4A-J** depicts several preferred embodiments of a catheter apparatus of use in configuration where the contrast agent delivery system and probes are catheter based;

**Figure 5A-D** depicts several preferred embodiments of a catheter apparatus of use in configuration where the contrast agent delivery system and probes are catheter based;

**Figure 6** depicts several preferred embodiments of a catheter apparatus of use in configuration where the contrast agent delivery system and probes are catheter based;

**Figures 7A-C** depicts several preferred embodiments of a catheter apparatus of use in configuration where the contrast agent delivery system and probes are catheter based;

**Figures 8A&B** depict a typical IVUS image and corresponding structures of a portion of a coronary artery;

**Figures 9A-C** depict three IVUS frames taken before microbubble injection (A), during injection or washout **(B)** and after injection (C);

**Figures 10A&B** depicts an average of a set of frames (A) used to produce an ROI mask (B), where the mask is indicated by white pixels; grey region contours are included for reference only;

**Figures 11A-D** depict plots of (**A**) $g_i$ for the first 201 frames of Case 1, (**B**) a frequency spectrum (DC component removed), (**C**) a filtered frequency spectrum, and (D) a resulting time-domain signal;

**Figures 12a-I** depicts IVUS frames from three case studies; **Figures 12A-C** depict Case 1 Frames 0, 444, and 972; **Figures 12D-F** depict Case 2 Frames 67, 2,713, and 6,283; **Figures 12G-I** depict Case 3 Frames 16, 445, and 1,010, where the first frame in each group is prior to, the second frame in each group is during, and the first frame in each group is after microbubble injection.;

**Figure 13** depicts a raw difference image for Case 1 data showing enhancements that are reported as a percentage of maximum enhancement (255) with negative values thresholded to 0%;

**Figures 14A&B** Case 1:Thresholded difference images: (a) during injection at frame 600, and (b) after injection at frame 800;

**Figure 15A&B** Case 1: Binary-thresholded difference images: (a) during injection at frame 600, and (b) after injection at frame 800. Light blue highlights plaque area;

**Figures 16A&B** Case 1: Enhancement for (a) intimo-medial/plaque, and (b) adventitial regions. Graph has been translated so the pre-injection mean is at 0%;

**Figures 17A&B** Case 1: Annotated enhancement for (a) intimo-medial/plaque, and (b) adventitial regions. Graph has been translated so the pre-injection mean is at 0%;

**Figures 18A&B** Case 2: Thresholded difference images: (a) during injection at frame 2,837, and (b) after injection at frame 5,308;

**Figures 19A&B** Case 2: Binary-thresholded difference images: (a) during injection at frame 2,837, and (b) after injection at frame 5,308. Light blue highlights plaque area;

**Figures 20A&B** Case 2: Enhancement for (a) intimo-medial/plaque, and (b) adventitial regions;

**Figures 21A&B** Case 2: Annotated enhancement for (a) intimo-medial/plaque, and (b) adventitial regions;

**Figures 22A&B** Case 3: Thresholded difference images: (a) during injection at frame 445, and (b) after injection at frame 815;

**Figures 23A&B** Case 3: Binary-thresholded difference images: (a) during injection at frame 445, and (b) after injection at frame 815. Light blue highlights plaque area;

**Figure 24** Case 3: Enhancement for intimo-medial/plaque region; and

**Figures 25A&B** Case 3: Annotated enhancement for (a) intimo-medial and (b) adventitial regions.

## DETAILED DESCRIPTION OF THE INVENTION

The inventors have found that an improved and system for imaging anatomy or morphology of a vessel and/or a tissue fed by vasa vasorum or other microvessels can be developed by analyzing data from acquired pre-contrast agent introduction and post-contrast agent introduction from such data acquisition techniques as intravascular ultrasound (IVUS) imaging, intravascular magnetic imaging, intravascular photonic imaging or other similar intravascular imaging techniques or the data acquisition technique can involve ultrasound; magnetic, terrahertz, microwave and/or photonic probes positioned to image a target, where the data images are transiently enhanced through an *in vivo* administration of an imaging contrast agent. Imaging data are collected before and after or before, during and after injection of the contrast agent into a vessel having a micro-catheter. In one preferred embodiment, the micro-catheter includes an appropriate probe held stationary in the vessel to be imaged and the exemplary method can also include a step of moving the probe within the vessel under controlled conditions and injecting the contrast agent at various intervals and with

various amounts so that data can be collected along a portion of the vessel. The inventors have also found that data from post-injection images or image frames show increased echogenicity in the vasa vasorum and in plaque resulting in enhanced areas within certain IVUS frames as analyzed through time. As time progresses, the enhanced areas fade. Generally, the entire data acquisition time (before, during and after) is between about 30 seconds and about 30 minutes. One preferred entire data acquisition time ranges from about 1 minute to about 20 minutes. Another preferred entire data acquisition time ranges from about 1 minute to about 15 minutes. Another preferred entire data acquisition time ranges from about 1 minute to about 10 minutes. Another preferred entire data acquisition time ranges from about 1 minute to about 5 minutes.

The present disclosure broadly relates to a method for acquiring and analyzing vessel and/or tissue and/or vasa vasorum data including the step of positioning a guiding catheter in a vessel in an animal including a human. Once positioned, a guide wire located within the guide catheter is extended into the vessel a desired distance. In one embodiment, a data collection probe is allowed to travel down the guide wire until it is situated adjacent a site to be analyzed in the vessel. In another embodiment, a probe is situated adjacent the site to be analyzed, a site that is supplied blood by the vessel and a contrast agent delivery system is allowed to travel down the guide wire until it is situated in a desired location in the vessel. After positioning the probe and/or the contrast agent delivery system, a pre-injection data sequence of the site is acquired on an intermittent, periodic, or continuous basis. An amount of an appropriate contrast agent is then introduced or injected into the vessel downstream, substantially at, and/or upstream of the probe position in the vessel in the first embodiment or at the location of the micro-catheter in the second embodiment. As the agent is being injected into the vessel, a during-injection data sequence of the site is then acquired on an intermittent, periodic, or continuous basis. After the injection of the contrast agent, a post-injection data sequence is acquired on an intermittent, periodic, or continuous basis. Preferably, the data sequences are collected on a continuous basis. Particularly, a single data sequence is collected and then divided into a pre-injection data sequence, a during-injection data sequence, and a post-injection data sequence. However, time continuity between the three sequences is not mandatory. Generally, the method also includes a saline injection step to flush any remaining contrast agent out of a contrast agent injection conduit into the vessel. After collection, the data are mathematically analyzed, compared and differenced to determine properties of the vasa vasorum, plaque, and/or vessel. Generally, the entire image collection period is between about 1.5 minutes and about 15 minutes, with each sequence representing a period of time between about 0.5 minutes and about 5 minutes, preferably, between about 1 and about 3 minutes. Besides obtaining data on a vessel, the method can also obtain information about desired tissue, cancer or organ sites fed by microvessels.

The present invention broadly also relates to a system for acquiring and analyzing intravascular data, where the system includes a guide-catheter which is adapted to be positioned within a vessel of an animal including a human. The guide-catheter includes one or more guide wires and one or more probes, contrast agent delivery assemblies, balloons or other devices housed within the guide-catheter or that can be fed through the guide-catheter, where the probes, contrast agent delivery assemblies, balloons or other devices are designed to travel down the guide wire forming different micro-catheter configurations. In one embodiment, the guide wire is designed to extend from a distal end of the guide-catheter a desired distance into the vessel and the micro-catheter components are designed to travel down the guide-wire until they are positioned adjacent a region, portion or segment of the vessel to be analyzed. In another embodiment, the probe is separate from the contrast agent delivery catheter and may be a probe associated with a second catheter or a probe positioned on a site external to an organ or tissue site such as a site of a skin, a site of an intestine, a site of a mouth, a site of an urinary tract, a site of a reproductive tract, a site external or internal to any desired target, but a site fed either directly or indirectly by the vessel into which the contrast agent is injection. The guide-catheter and/or the micro-catheter include an outlet associated with a conduit extending from the outlet to a contrast agent delivery assembly having a pump, where the assembly is adapted to inject a desired amount of a contrast agent from a storage vessel through the conduit and out of the outlet and into the vessel. The probe is adapted to acquire data about the region, portion or site on an intermittent, periodic or continuous basis. The acquired data are sent down a data collection conduit extending from the probe to a data collection and data analyzer unit situated outside the body of the animal including the human and stored. Preferably, the system is adapted to acquire three sequences of data: (1) a data sequence acquired for several minutes prior to contrast agent injection to produce a pre-injection data sequence, (2) a data sequence acquired during contrast agent injection to produce a during-injection data sequence, which is generally acquired approximately one minute after normal saline is inj ected into the conduit to flush out remaining contrast agent, because the injection of the contrast agent can temporarily washing out any signal due to an echo-opacity of a lumen as it is being saturated with the contrast agent; and (3) a data sequence acquired for several minutes after saline injection to produce a post-injection data sequence. These three data sequences are then analyzed and vessel and/or vasa vasorum and/or plaque and/or tissue properties are determined and/or quantified.

One aspect of the present disclosure is a microbubble-based vessel and vasa vasorum imaging technique to quantify vasa vasorum density which serves as a proxy of macrophage density, to quantify plaque density and/or to quantify plaque inflammation by comparing ultrasound signals, images or frames before and after injection of microbubble ultrasound contrast agents or preferably before, during and after such an injection. Although ultrasound is the preferred

detecting format, magnetic and photonic formats can also be used with the contrast agent being a magnetic contrast agent in the case of a magnetic format and a photonic contrast agent in the case of a photonic format. Moreover, a combination of detecting formats can be used as well. Thus, an ultrasound and magnetic probe can used simultaneously with a mixed contrast agent including an ultrasound contrast agent and a magnetic contrast agent.

Another aspect of the present disclosure is a contrast enhanced luminology method performed by improving edge detection and clarifying an interface between blood and lumen and also between different layers of an artery, especially a coronary artery, and interfaces between vessels or micro-vessels.

Another aspect of the present disclosure is a microbubble contrast enhanced plaque detection and characterization using signal intensity, spatial domain and frequency domain analyses to enhance current virtual histology visualization. Also intraplaque hemorrhage or presence of fissure in a plaque cap, or leakage angiogenesis can be identified by imaging microbubbles in plaque. Unlike circulating microbubbles in vasa vasorum, these microbubbles get stuck in the plaque. In case of ruptured plaque, one can image flow and turbulence going inside the plaque. Although ultrasound is the preferred detecting format, magnetic and photonic formats can also be used with the contrast agent being a magnetic contrast agent in the case of a magnetic format and a photonic contrast agent in the case of a photonic format.

Another aspect of the present disclosure is a microbubble dilution flowmetry technique. The technique involves injecting microbubbles to create a transient change in signal intensity of blood near the IVUS catheter and plotting the signal intensity against time to measure blood flow. Although this flow is affected by the presence of the catheter, with proper calibration, calculation and simulation, the actual blood flow at the catheter location can be determined as if the catheter were not present. Similarly, a change in blood flow can be measured. This data can be used to measure fractional flow reserve or other flow related physiologic properties. The point of sampling from which the signal is measured can be expanded to create a cross-sectional flow map of the area, which can be color coded. Such a microbubble-based flow measurement and imaging technique can also show shear stress and flow patterns in coronary arteries. Although such a pattern is not natural and is disturbed by the catheter, such data can be useful in certain circumstances, for example after placing a stent, images can be acquired to determine an interaction of the stent with the blood. Although ultrasound is the preferred detecting format, magnetic and photonic formats can also be used with the contrast agent being a magnetic contrast agent in the case of a magnetic format and a photonic contrast agent in the case of a photonic format.

Another aspect of the present disclosure is a microbubble enhanced visualization of stent positioning and deployment methodology to reduce or prevent mal-positioning of the stent. The present microbubble based IVUS imaging methodology can be used help interventional cardiologists in placing stents or examining proper deployment of stents especially in complicated cases. Although ultrasound is the preferred detecting format, magnetic and photonic formats can also be used with the contrast agent being a magnetic contrast agent in the case of a magnetic format and a photonic contrast agent in the case of a photonic format.

Another aspect of the present disclosure is a microbubble enhanced coronary strain and shear stress imaging methodology. The present IVUS with microbubble imaging methodology can be used to help in edge detection which is critical in stress/strain imaging. Although ultrasound is the preferred detecting format, magnetic and photonic formats can also be used with the contrast agent being a magnetic contrast agent in the case of a magnetic format and a photonic contrast agent in the case of a photonic format.

Another aspect of the present invention is a microbubble dispensing catheter for the controlled release of microbubbles, where the microbubble dispensing portion of the catheter is associated with the micro-catheter, the guide-catheter or with both so that a controlled release of microbubbles can be effectuated. Although ultrasound is the preferred detecting format, magnetic and photonic formats can also be used with the contrast agent being a magnetic contrast agent in the case of a magnetic format and a photonic contrast agent in the case of a photonic format.

Another aspect of the present disclosure is a endothelial dependent and independent coronary vasoreactivity software methodology. With microbubble-based flow measurement and enhanced luminology of this invention, endothelial dependent and independent vasoreactivity can be measured with IVUS catheter. An IVUS-based technique has already been disclosed by others, but the enhancement achieved by the present invention using only a single catheter and not two catheters one a Doppler or thermal dilution flow catheter is novel. Although ultrasound is the preferred detecting format, magnetic and photonic formats can also be used with the contrast agent being a magnetic contrast agent in the case of a magnetic format and a photonic contrast agent in the case of a photonic format.

Another aspect of the present disclosure is imaging endothelial function in both epicardial coronary and microvascular level. Currently, there is no software or clinically available tool that would enable measurement of endothelial function. The present invention includes a user-friendly software tool to implement vasoreactivity screening in catheter labs. Such software will overlap the endothelial function measurement over the IVUS imaging and microbubble enhanced images to create a comprehensive diagnostic package to enhance IVUS screening procedures. Although ultrasound is the preferred detecting format, magnetic and photonic formats can also be used with the contrast agent being a magnetic contrast agent in the case of a magnetic format and a photonic contrast agent in the case of a photonic format.

Another aspect of the present disclosure is to use high-frequency, real-time low mechanical index images to

measure retained microbubbles in the plaque, where endothelial dysfunction is associated with increased permeability of an arterial wall.

In addition to using pharmachological stimuli for measuring coronary vasoreactivity and epicardial endothelial function, one can use a simple cold pressure test which causes vasodilation in normal coronary arteries, but constricts the artery in patients with cardiovascular risk factors. This can be done by immersing patients hand in cold water making Ultimate IVUS screening less complicated and more reproducible. Although ultrasound is the preferred detecting format, magnetic and photonic formats can also be used with the contrast agent being a magnetic contrast agent in the case of a magnetic format and a photonic contrast agent in the case of a photonic format.

Another aspect of the present disclosure is to targeted microbubbles delivery and imaging against thrombus and other targets to enhance the specificity of microbubble imaging for vulnerable plaque detection. Another aspect of the present invention is to targeted microbubbles delivery and imaging against annexin V to provide apoptosis imaging, which can be useful in prediction of plaque rupture.

Another aspect of the present disclosure is intra-pericardial microbubble injection for both enhanced contrast delivery and drug delivery. Intra-pericardial fluid have a much longer contact time with plaque than intraluminal, therefore, intra pericardial delivery of microbubbles either through arterial access or through an atrial appendage can be used to access such plaques.

Another aspect of the present invention is to create an advanced IVUS-based protocol for screening of vulnerable patients in catheter labs. IVUS techniques currently are used in less than 10% of interventional procedures. The IVUS protocol of this invention utilizes various features of related IVUS technologies to create a cumulative risk index. The protocol can be used to assess and/or predict a total vulnerability of coronary arterial trees. In this protocol, endothelial function and vasoreactivity, which is based on pharmacological intervention, are major components. The protocol involves imaging all three coronary arteries, right, left and circumflex, using the IVUS imaging methodology of this invention. Endothelial function is measured both in areas with and without endothelial dysfunction. Although ultrasound is the preferred detecting format, magnetic and photonic formats can also be used with the contrast agent being a magnetic contrast agent in the case of a magnetic format and a photonic contrast agent in the case of a photonic format.

The IVUS protocol of this invention can supplement other risk assessment protocols. Basically, a patient is initially screening by various non-invasive and serum marker risk assessment protocols such as the Framingham Score, CRP, non-invasive endothelial function measurement, non-invasive coronary CT imaging or other non-invasive protocols. If warranted, the patient is then screened using the IVUS protocol of this invention. The protocol of this invention is thus an advanced screening technique for all vulnerable patients, patients whose non-invasive risk factors indicate endothelial dysfunction, early stage atherosclerosis, moderate stage atherosclerosis or advanced stage atherosclerosis. The IVUS protocol of this invention can also be used to screen subjects at risk of future heart attacks.

Using the IVUS protocol microbubbles in the lumen can be used to measure fibrous cap thicknesses by mathematically subtracting or differencing before and after injection images particularly in areas having some degree of fiber formation or calcification. The vasa vasorums usually do not reach fibrous caps, and, therefore, subtracting the before and after injection images will allow the determination of a thickness of a fibrous cap.

Using the IVUS software and the IVUS protocol, a total risk or vulnerability index for a whole coronary artery, artery tree or arteries can be determined instead of a single plaque associated with a single artery. Using a combination of data from non-invasive procedures and vessel and vasa vasorum data and cardiac flow data derived from the IVUS of this invention, a more effective total risk assessment scale of cardiac patients can be constructed. The new risk assessment scale includes data concerning calcification in areas of the coronary arteries, where proximal calcification gives rise to a greater total risk value than distal calcification. Moreover, plaques or calcifications in different areas of coronary branches will produce different total risk values. For example, patients with short left coronary artery trunk will be assessed with a higher total risk. The total risk scale is generated by weighting the data value from non-invasive and invasive procedures. The exact weighting can be adjusted as more data are collected and more consequences are gathered to produce a more accurate risk assessment model. Thus, the model of this invention involves the combination of non-invasive data and data derived by the IVUS methods of this invention to construct an improved risk assessment valuation of at risk patients.

The present disclosure also relates to imaging tissue sites with any one of the techniques described above, but with the added feature of exposing the area being analyzed to energy designed to either vibrate the contrast agent or in the case of microbubbles to vibrate and/or rupture the microbubbles. The feature can be a sonic probe operated at a frequency designed either to cause the contrast agent to move periodically at a known frequency or to cause the contrast agent to disappear. For microbubbles, the vibration can be induced by exposing the site to a lower frequency of sound waves, generally, in the low megahertz range from about 0.5 MHz to about 10 MHz at an intensity sufficient to vibrate the microbubbles, but not sufficient to rupture the microbubbles. On the other hand, to rupture the microbubbles, generally only the intensity of the sonic waves must be change - higher intensity causes rupture, or the frequency can be increased to a frequency at which the microbubbles will burst. The use of a vibratory and/or contrast agent destruction devices is designed to increase signal intensity and signal change over time so that more information about the tissue

site can be ascertained.

The methods although preferably use non-native contrast agents, can be adapted to use native carriers as the contrast agent. One preferred method uses read blood cells as the contrast agent. The method operates by positioning a probe at a tissue site to be analyzed. A catheter equipped with a balloon is positioned in a vessel associated with the site. A first data sequences is acquired. The balloon is then inflated to alter a flow of blood to the site. A second data sequence can be acquired during the flow alteration. Then, the flow is returned is altered again and a third data sequence is acquired. The data sequences are then analyzed as set forth herein. One preferred sequence for performing this method is to acquire the data continuously from a time before the balloon is inflated completely stopping blood flow, during the blood flow stoppage period, and for a time after balloon deflation. Alternatively, the method can be performed by stopping blood flow, acquiring a first sequence during the stoppage period. The balloon is then deflated and a second data sequence is acquired. Again, the data sequences are then analyzed for enhancements as set forth herein.

Another method using cells as the contrast agent is to treat cells with nanoparticles known to be absorbed or taken up by cells and injection the modified cells into a vessel feeding the tissue site to be analyzed. Thus, red blood cells can be treated with magnetic, near infrared, terrahertz, microwave or other contrast agents or mixtures thereof and injected into a vessel either feeding the tissue site or draining the tissue site.

Besides analyzing tissue sites for anatomical, morphological and/or functional features, the present methods can also be used to monitor therapies that involve tissue or cell transplantation. For example, certain new treatment of damaged organs or tissues involves the use of stem cells. Unfortunately, such treatment are wrought with considerable inefficiencies in the incorporation of the stem cells within the site of transplantation. The present methods can be used to monitor and/or post transplantation to ascertain the degree of stem cell incorporation. Thus, a probe can be positioned adjacent to a transplantation site to be analyzed and a degrees of stem cell incorporation can be determined. In the case of cardiac stem cell implantation, the methods of this invention can be used to ascertain an extent of vascularization of heart tissue that have been implanted with the stem cells. Moreover, to increase detection, the stems cells can be treated with a nanoparticle contrast agent. This same method can be used in tissue or organ transplantation to assay the degree of integration of the new organ or tissue into the animal body and to ascertain a degree of rejection.

It should be recognized that in all of these methods where the data is acquired on a continuous basis throughout the entire sequence of events, the data is later divided into the pre; during and post contrast agent sequences based on the time each step in the method is performed.

## New Catheter Designs

Although the IVUS methods of this invention can be carried out with current catheters, the IVUS methodology of this invention can be more effectively implemented with new catheter designs tuned to improve contrast agent enhanced characterizations of structures such as vessels, vasa vasorum, plaques and/or other structures. One class of improved catheter designs include improved microbubble or other contrast agent controlled delivery systems. Not only can these delivery systems be used to produce a controlled introduction of contrast agents, the delivery systems can also be used to delivery a controlled amount of a therapeutic agent.

One preferred embodiment of a catheter assembly of this invention includes a guide-catheter and a micro-catheter housed therein. The guide-catheter includes a microbubble storage and delivery system having a microbubble outlet located at or near a distal end of the guide-catheter. The micro-catheter includes an ultrasound probe, where the micro-catheter has an aperture therethrough and is designed to be fitted onto a guide wire and travel down the guide-wire until the micro-catheter is positioned adjacent a target site of a vessel.

Another preferred embodiment of a catheter assembly of this invention includes a guide-catheter, a micro-catheter and a microbubble conduit housed therein. The guide-catheter includes a microbubble storage and delivery system located at or near its distal end. The micro-catheter includes an ultrasound probe and a microbubble outlet, where the microbubble conduit connects the microbubble storage and delivery system to the microbubble outlet. Again, the micro-catheter has an aperture therethrough and is designed to be fitted onto a guide wire and travel down the guide-wire until the micro-catheter is positioned adjacent a target site of a vessel.

Another preferred embodiment of a catheter assembly of this invention also includes a guide-catheter and a micro-catheter housed therein. The guide-catheter includes a microbubble storage and delivery system having a micro-bubble outlet located at or near its distal end and an inflatable balloon located at or near its distal end and designed to alter blood flow into the vessel. The micro-catheter includes an ultrasound probe. Again, the micro-catheter has an aperture therethrough and is designed to be fitted onto a guide wire and travel down the guide-wire until the micro-catheter is positioned adjacent a target site of a vessel.

Another preferred embodiment of a catheter assembly of this invention also includes a guide-catheter, a micro-catheter and a microbubble conduit housed therein. The guide-catheter includes a microbubble storage and delivery system located at or near its distal end and an inflatable balloon located at or near its distal end and designed to alter blood flow into the vessel. The micro-catheter includes an ultrasound probe and a microbubble outlet, where the micro-

bubble conduit connects the microbubble storage and delivery system to the microbubble outlet. Again, the micro-catheter has an aperture therethrough and is designed to be fitted onto a guide wire and travel down the guide-wire until the micro-catheter is positioned adjacent a target site of a vessel.

Another preferred embodiment of a catheter assembly of this invention also includes a guide-catheter and micro-catheter housed therein. The guide-catheter includes a microbubble storage and delivery system having a micro-bubble outlet located at or near its distal end. The micro-catheter includes an ultrasound probe and a Doppler probe. Again, the micro-catheter has an aperture therethrough and is designed to be fitted onto a guide wire and travel down the guide-wire until the micro-catheter is positioned adjacent a target site of a vessel.

Another preferred embodiment of a catheter assembly of this invention also includes a guide-catheter, a micro-catheter and a microbubble conduit housed therein. The guide-catheter includes a microbubble storage and delivery system located at or near its distal end. The micro-catheter includes an ultrasound probe, a Doppler probe and a microbubble outlet, where the microbubble conduit connects the microbubble storage and delivery system to the micro-bubble outlet. Again, the micro-catheter has an aperture therethrough and is designed to be fitted onto a guide wire and travel down the guide-wire until the micro-catheter is positioned adjacent a target site of a vessel.

Another preferred embodiment of a catheter assembly of this invention also includes a guide-catheter and a micro-catheter housed therein. The guide-catheter includes a microbubble storage and delivery system having a micro-bubble outlet located at or near its distal end and an inflatable balloon located at or near its distal end and designed to alter blood flow in the vessel. The micro-catheter includes an ultrasound probe and a Doppler probe. Again, the micro-catheter has an aperture therethrough and is designed to be fitted onto a guide wire and travel down the guide-wire until the micro-catheter is positioned adjacent a target site of a vessel.

Another preferred embodiment of a catheter assembly of this invention also includes a guide-catheter, a micro-catheter and a microbubble conduit housed therein. The guide-catheter includes a microbubble storage and delivery system located at or near its distal end and an inflatable balloon located at or near its distal end and designed to alter blood flow in the vessel. The micro-catheter includes an ultrasound probe, a Doppler probe and a microbubble outlet, where the microbubble conduit connects the microbubble storage and delivery system to the microbubble outlet. Again, the micro-catheter has an aperture therethrough and is designed to be fitted onto a guide wire and travel down the guide-wire until the micro-catheter is positioned adjacent a target site of a vessel.

Another preferred embodiment of a catheter assembly of this invention also includes a guide-catheter and a first micro-catheter and a second micro-catheter housed therein. The guide-catheter includes a microbubble storage and delivery system located at or near its distal end. The first micro-catheter includes an ultrasound probe. The second micro-catheter includes an inflatable balloon. Again, the micro-catheters have apertures therethrough and is designed to be fitted onto guide wires and travel down the guide-wires until the probe micro-catheter is positioned adjacent a target site of a vessel and the balloon micro-catheter is positioned upstream, at or downstream of the probe micro-catheter to alter blood flow through the vessel.

Another preferred embodiment of a catheter assembly of this invention also includes a guide-catheter and a first micro-catheter and a second micro-catheter and a microbubble conduit housed therein. The guide-catheter includes a microbubble storage and delivery system located at or near its distal end. The first micro-catheter includes an ultrasound probe and a microbubble outlet, where the microbubble conduit connects the microbubble storage and delivery system to the microbubble outlet. The second micro-catheter includes an inflatable balloon. Again, the micro-catheters have apertures therethrough and is designed to be fitted onto guide wires and travel down the guide-wires until the probe micro-catheter is positioned adjacent a target site of a vessel and the balloon micro-catheter is positioned upstream, at or downstream of the probe micro-catheter to alter blood flow through the vessel.

Another preferred embodiment of a catheter assembly of this invention also includes a guide-catheter and a first micro-catheter and a second micro-catheter housed therein. The guide-catheter includes a microbubble storage and delivery system located at or near its distal end. The first micro-catheter includes an ultrasound probe and a Doppler probe. The second micro-catheter includes an inflatable balloon at or near its distal end. Again, the micro-catheters have apertures therethrough and is designed to be fitted onto guide wires and travel down the guide-wires until the probe micro-catheter is positioned adjacent a target site of a vessel and the balloon micro-catheter is positioned upstream, at or downstream of the probe micro-catheter to alter blood flow through the vessel.

Another preferred embodiment of a catheter assembly of this invention also includes a guide-catheter and a first micro-catheter and a second micro-catheter and a microbubble conduit housed therein. The guide-catheter includes a microbubble storage and delivery system located at or near its distal end. The first micro-catheter includes an ultrasound probe, a Doppler probe and a microbubble outlet. The second micro-catheter includes an inflatable balloon. Again, the micro-catheters have apertures therethrough and is designed to be fitted onto guide wires and travel down the guide-wires until the probe micro-catheter is positioned adjacent a target site of a vessel and the balloon micro-catheter is positioned upstream, at or downstream of the probe micro-catheter to alter blood flow through the vessel.

Another preferred embodiment of a catheter assembly of this invention also includes a guide-catheter and a first micro-catheter and a second micro-catheter and a third micro-catheter housed therein. The guide-catheter includes

a microbubble storage and delivery system having a microbubble outlet located at or near its distal end. The first micro-catheter includes an ultrasound probe located at or near its distal end. The second micro-catheter includes a Doppler probe also located at or near its distal end. The third micro-catheter includes an inflatable balloon at or near its distal end. As in other embodiment, the catheter assembly can also include a microbubble conduit and the microbubble outlet can be located at or near a distal end of first and/or second micro-catheter.

Another preferred embodiment of a catheter assembly of this invention also includes a guide-catheter and a micro-catheter housed therein. The micro-catheter includes an ultrasound probe, a microbubble storage and delivery system having a microbubble outlet, and an inflatable balloon located at or near its distal end. The micro-catheter can also include a secondary probe such as a Doppler probe.

It should also be recognized that the Doppler probe can be replaced by other probes or that other probes can be included in the any of the designs described above.

In some of these designs, the guide-catheter includes an extra lumen to deliver microbubbles at a certain distance from the probe of the micro-catheter, which allows on demand delivery of microbubbles. Such catheters along with imaging software of this invention can utilize microbubble-dilution flowmetry techniques to measure intracoronary blood flow. More importantly, these catheters can have a built in distal balloon or a double-balloon that can be inflated to a low pressure to increase a time and pressure for microbubble profusion inside a coronary artery to improve or change the rate at which the vasa vasorum is filled with microbubbles. Because the vasa vasorum is easily compressed and increased intraluminal pressure causes incomplete or poor delivery of the microbubbles into the vasa vasorum, a catheter able to control the delivery of microbubbles and to control the rate of profusion into the vasa vasorum would be a particular advantage and advancement of the art.

The catheters' designs of this invention also make possible the collection of diagnostic data, the administration of therapeutic agents and the analysis of therapeutic agent effects in a single catheter. For example, the catheter can trigger microbubble-based drug delivery to an area of interest. The catheters of this invention can use various ultrasound wave delivery and imaging protocols including Doppler, Harmonic, FLASH pulse, high intensity ultrasound etc. Catheters including ultrasound probes and any of these secondary probes can help provide enhanced imaging as well as controlled destruction of microbubbles and the release of any microbubble based drugs. Unlike drug-eluting stents, the catheters of this invention can deliver antiinflammatory, anti-proliferative, anti-angiogenesis and/or anti-atherosclerotic microbubble based drugs to multiple plaques and/or sites of coronary arteries.

The present invention can use any type of microbubbles including microbubbles of different sizes, ultrasound reflection and/or absorption properties and half-lives. Because microbubble flow into the vasa vasorum and plaques is slow compared to flow into and through the coronary arteries, the inventors believe that microbubbles with properties to allow longer imaging time frames would be desirable for maximizing vasa vasorum and plaque visualization.

Having the ultrasound probe (scanner) located close to a microbubble delivery outlet (in case of coronary applications) provide opportunities to develop novel harmonic and Doppler shift imaging of the vasa vasorum and plaques. Moreover, using microbubbles for plaque characterization can provide new opportunities for using high-resolution / high-frequency ultrasound to improve data collection and data analysis.

The probes of the present invention for use with catheters or to be located external to the site to be analyzed can also include variable frequency transmitter and detector elements, which can be within the same structure or within separate structures. Thus, the probes can simultaneously image the system within one frequence domain, while exposing the site to a second or multi-frequency domains. The other frequency domains can be used to induced a period oscillation of a position of a contrast agent, to rupture or disperse the contrast agent or to do both oscillate and disintegrate the contrast agents.

It should also be recognized that for ultrasound analyses, the resolution is not a the single microbubble level, but at a collection of microbubbles level. Thus, when the microbubbles or any other micrometer or nanometer sized contrast agent are injected, the probe detects a perfusion or flow of the agents into the tissue site being analyzed, until the concentration of the contrast agent drops below a detection threshold, which differs for each contrast agent used and for each probe used as is well known in the art.

The probes of this invention can also be designed with the capability of acquiring harmonic data as well as the frequency and intensity data.

While one preferred contrast agent delivery system for use in the methods of this disclosure are associated with a catheter and comprise a storage unit and a pumping unit, the delivery system can also be a needle connected to a pump or a micro-pump assembly, where the needle is inserted into an artery or vein associated with the site to be analyzed or the assembly is inserted into an artery or vein associated with the site to be analyzed. The use of a needle or a micro-pump is especially suitable for use with probes that are situated external to a vessel supplying or removing blood from the site to be analyzed. Thus, if the probe is positioned at a site within the colon, i.e., in contact with a site of the lining of colon and the needle is used to injection microbubbles into an artery supply blood to the site or a vein removing blood from the site so that a pre- and post data sequence can be acquired.

Another preferred contrast agent delivery modality, is to administer the contrast agent throughout the entire

circulatory system either intra-arterially or intravenously. In this modality, the probes detects the contrast agent as it enters and leaves the site being analyzed.

**Contrast Agents**

Although microbubbles are currently the preferred ultrasound contrast agent, any agent that can be introduced into a vessel and enhance or augment an ultrasound response is suitable for use with this invention. Preferred microbubbles have diameters in the micron or nanometer size scale. If other types of contrast agents are used, then they too should have particle size diameters in the micron and sub-micron ranges. Generally, the size of the contrast agents should be less than or equal to the diameter of a red blood cell. Preferred ranges are from about 3 to about 0.01 microns or from about 10 to about 3000 nanometers; 10 to about 2000 nanometers; 10 to about 1000 nanometers; and 10 to about 500. Of course, larger and smaller size can be used as well, with smaller size being preferred because smaller sized particles can profuse more readily into sites such a vasa vasorum, plaques or other sites with low flow characteristics.

Other contrast agents include microbubbles that are magnetically active for use with magnetic probes, microbubbles that are near Infrared active for use with near Infrared probes, microbubbles that are terrahertz active for use with terrahertz probes, microbubbles that are microwave active for use with microwave probes, microbubbles visible with protonic probes, or microbubbles that have components that are visible by one or more probe types.

Yet other contrast agents include nanoparticles that are magnetically active for use with magnetic probes, nanoparticles that are near Infrared active for use with near Infrared probes, nanoparticles that are terrahertz active for use with terrahertz probes, nanoparticles that are microwave active for use with microwave probes, nanoparticles visible with protonic probes, or nanoparticles that have components that are visible by one or more probe types.

Yet other contrast agents include nanoparticles that are designed to be absorbed into cells such as stem cells, red or white blood cells, transplant cells, or other cell types, where the nanoparticles include nanoparticles that are magnetically active for use with magnetic probes, nanoparticles that are near Infrared active for use with near Infrared probes, nanoparticles that are terrahertz active for use with terrahertz probes, nanoparticles that are microwave active for use with microwave probes, nanoparticles visible with protonic probes, or nanoparticles that have components that are visible by one or more probe types.

It should also be recognized that vasa vasorum or any other vascularization that supplies blood to tissue sites in an animal including a human, the vessels form a network of ever finer vessels down to the capillary size. Some contrast agents may not be able to pass through there capillaries. The present invention can be used to map the network, by changing a size of the contrast agent provided that the contrast agent will eventually disintegrate or can be disintegrated as previously discussed. Thus, the size of the capillaries and the density of the capillaries associated with the site can be determined. This technique may be ideally suited for determining the density and characteristics of tumor vascularization or stem cell implantation vascularization.

**Method Utility**

The methods can be used to measure at least the following attributes of a vessel and the vasa vasorum associated therewith: (1) presence or density of vasa vasorum;(2) vasa vasorum circulation; (3) leakage of vasa vasorum and (4) presence and density of plaques. In addition, the methods can be used to measure flow characteristics into a vessel and especially into the vasa vasorum associated therewith, to monitor vasa vasorum and vessel responses to various stimuli and to monitor the response of the vasa vasorum and plaque to different treatment protocols.

The present methods can be used to measure the presence and/or density of vasa vasorum by comparing differences in intensity and/or frequency of IVUS images before and after microbubble injection or before, during, and after microbubble injection.

The present methods can be used to measure vasa vasorum circulation using Doppler IVUS or by measuring changes in intensity of the IVUS images over a period of time. The measurements are an indicator of flow in the vasa vasorum or perfusion into plaque. Additionally, the data may give an indirect measure of plaque stiffness, i.e., movement of microbubbles in the plaque as a function to time due to factors such as compression of the plaque during each cardiac cycle, *etc.*

The present methods can be used to measure leakage of vasa vasorum by comparing the intensity of IVUS images before injection and long after injection, where the period after should be long enough so that minimal or no microbubble are still flowing through vasa vasorum. Flow into vasa vasorum typically ranges from an average of about 10 and 30 times slower than flow into the coronary arteries. Therefore, given a normal flow of 100-120 mL/second in the coronary artery, one would expect that 5 cc microbubbles injected intracoronary to last no more than between about 2 and 3 minutes. This time may vary per position in an artery, but overall after a safe period of time in which microbubbles are still stable (within their half-life), one can compare the image with the baseline and attribute the difference in intensity to microbubbles profusion into plaque or trapped in clogged-up vessels or vasa vasorum.

The methods and catheters can be used to help in stent placement and deployment reducing or eliminating incorrect stent placement and stent deployment, especially in stents that define one or more struts.

Using catheters equipped with Doppler probes or other motion sensitive ultrasound based probes, one can image coronary artery lumen and subsequently vasa vasorum in atherosclerotic plaques and also pericardial area. Preferably, microbubbles used in such applications should preferably have longer half lives than ordinary microbubbles, which are used predominantly for intraluminal perfusion imaging.

The vasa vasorum in a normal artery and in an inflamed or wounded artery, e.g., arteries including atherosclerotic plaques, should have different roles and significance and such differences can be ascertained and quantified by the method.

The issue of a smooth boundary layer comprising only microbubbles or only blood, is not thought to be a big concern due to the pulsative nature of flow, the torturous anatomy and rapid spiral motions of the flow into the arteries during each cardiac cycle, especially when a catheter that occupies about half of the artery in present in the artery. However, from a fluid mechanics point of view such flows may cause a significant blackout periods after microbubble injection, a microbubble injection methodology that would insure a more homogenous injection of microbubbles is likely to reduce or eliminate any boundary layer effects. Because most coronary vasa vasorum originate from proximal branch points and enter into adventitia, a preferred microbubble delivery system should be capable of delivering an equal amount of microbubbles to each branch that feed a particular vasa vasorum. Thus, the catheters of this invention including a controlled microbubble delivery system that can deliver microbubbles independent of the guide-catheter is capable of delivering an exact amount of microbubbles to a proximal segment of a plaque. Alternatively, an internal control (the total density or sound scattering induced by the passage of microbubbles in the lumen) can be detected and analyzed. Such data can be used as a surrogate measure of the exact mixture of blood and microbubbles and can be entered as a coefficient or factor into the image analysis and plaque perfusion calculations of this invention. Thus, a 20% microbubble + 80% blood mixture will give a different enhancement than a 80% microbubble + 20% blood mixture. By measuring IVUS images at different microbubble to blood mixtures, more information about flow profiles into vasa vasorum and plaques can be obtained.

## Saline Contrast Enhancement Effects

Fast injection of normal saline can create a significant darkening contrast effect that can be used in IVUS imaging, particularly in detecting boundaries such as lumen boundaries. Thus, by injecting a large bolus of saline (*e.g.,* 10 cc) fast a significant contract enhancement effect can be measured.

Imaging of vasa vasorum in coronary arteries can be accomplished by other non ultrasound contrast agents and probes such as a near infrared (NIR) spectroscopy catheter in conjunction with a near infrared contrast agent. Also an intravascular magnetic sensor can be used with magnetic contrast agents. A simple example of NIR based approach is OCT and NIR spectroscopy catheter. The contrast agent can be a dye having a strong NIR signature. Lipophilic agents are preferred.

It has been known that chronic hyperlipidemia is associated with endothelial dysfunction even in the absence of atherosclerosis. It has also been shown that a change in electrostatic charge of abnormal endothelium is capable of causing adherence of albumin-coated microbubbles. Endothelial dysfunction is associated with increased retention of microbubbles. This can provide additional value for microbubble ultrasound imaging of vulnerable plaques. Microbubbles seem to have a tendency to attach to the endothelial cells either on the surface of the plaque or inside the vasa vasorum.

The vasa vasorum imaging method can be combined with a intracoronary magneto sensor and coronary angiography visualization system to further enhance data collecting and image integrity. Such a combined system would allow a cardiologist to use an X-ray opaque catheter with X-ray visible markers and through controlled pull-back and the use of imaging fusion software identify the area of magnetic contrast enhancement. Also, based on change of magnetic signals over time one can measure the perfusion rate of the plaque.

The present imaging methodology can also be used to monitor certain therapies such as cancer therapy where the cells in the cancerous growth are susceptible to microbubble to other contrast agent profusion. Thus, the imaging technique of this invention can be used to monitor chemo-therapy, radiation therapy or other therapies directed to treat cancers of the esophagus, the prostrate, the bladder, the stomach, the large and small intestines, the mouth, the vagina, the cervix, the fallopian tube, the ovaries, the uterus, or other organs to which a probe can be positioned external to the organ.

Using the software of this invention, one can monitor tumor response to cancer therapy particularly to anti-angiogenesis therapy. Ofcourse, this can be used for non-invasive monitoring of tumors with lower frequency that can also use Harmonic imaging.

The present invention can be used to image inflammation using ultrasound or other contrast capable techniques, where the contrast agent is fed through a vessel into inflamed site and a probe situated adjacent the site takes data before contrast agent injection and after contrast agent injection or before, during and after contrast agent injection. The

collected data is then analyzed to determine regions of enhancement and correlating the regions of enhancement with the pathology of the inflammation process.

## DETAILED DESCRIPTION OF A PREFERRED MEETHOD OF THIS INVENTION

Referring now to **Figures 1A-F**, a catheter assembly, generally **100**, includes a guide-catheter **102** and a micro-catheter **104** having an magnetic probe **106** at its distal end **108**. Looking now at **Figure 1B,** the guide-catheter **102** is introduced into a peripheral artery such as a femoral artery and snaked into and through the aorta **110** and into a coronary artery **112**. Once the guide-catheter **102** is properly positioned, guide wire **114** extended into the artery **112** and allowed to travel a desired distance into the artery **112**. The micro-catheter **104** including the probe **106** is allowed to travel down the guide wire **114** to a desired location, generally a location including a plaque **116**. Once the probe **106** is properly situated, a series of images are taken and then an amount of a contrast agent is injected into the artery **112.** A series of images are then taken. If a saline flush is used, then a given amount of saline is injected to flush out any remaining microbubble contrast agent in the supply systems. Finally, a series of images are taken after microbubble and saline injection (if done). After one location is analyzed, the micro-catheter can be repositioned and a second site in the artery **112** can be analyzed. The plots in the Figures show where the data was taken in the artery and the difference between a normal artery and a diseased artery.

Referring now to **Figures 2A-C**, the three types of vasa vasorum are shown. Looking at **Figure 2A**, a vasa vasorum intema (WI), **Vasa Vasorum Interna (WI)**, is shown associated with an arterial wall, **Wall,** of an arterial lumen, **Artery Lumen.** Looking at **Figure 2B**, a vasa vasorum externa (VVE), **Vasa Vasorum Interna (WE)**, is shown. Looking at **Figure 2C**, a venous vasa vasorum (WV), **Venous Vasa Vasorum (WV)**, is shown coming from a proximally located vein, **Vein.**

Referring now to **Figures 3A-I**, a longitudinal view and eight cross-sectional views of a diseased arteries are shown illustrating the different types of vulnerable plaque. Looking at **Figure 3A**, coronary artery **300** is shown having a normal section **302**, a rupture-prone plaque section **304**, a ruptured plaque section **306**, an erosion-prone plaque section **308**, an eroded plaque section **310**, an intraplaque hemorrhage section **312**, a calcific nodule section **314,** and a chronically stenotic plaque section **316**. Looking at **Figure 3B**, a cross-section of the normal section **302** is shown to include the lumen **318**, the tunica intema **320**, the tunica media **322**, and the tunica externa **324.** Looking at **Figure 3C**, a cross-section of rupture-prone plaque section **304** is shown to include a large lipid core **326**, a thin fibrous cap **328** infiltrated by macrophages **330** and collagen **332**. Looking at **Figure 3D**, a cross-section of the ruptured plaque section **306** is shown to include anon-occlusive clot or subocclusive thrombus **334** and an early organization including a ruptured cap **336.** Looking at **Figure 3E**, a cross-section of the erosion-prone plaque section **308** is shown to include a proteoglycan matrix **338** in a smooth muscle cell-rich plaque **340** evidencing a dsyfunctional endothelium **342** including platelets **344.** Looking at **Figure 3F**, a cross-section of the eroded plaque section **310** is shown to include a subocclusive thrombus or non-occlusive mural thrombus / fibrin **346.** Looking at **Figure 3G**, a cross-section of the intraplaque hemorrhage section **312** is shown to include an intact cap **348** and a leaking vasa vasorum **350.** Looking at **Figure 3H**, a cross-section of the calcific nodule section **314** is shown to include a calcific nodule **352** protruding into the vessel lumen **318**. Looking at **Figure 3I,** a cross-section of the chronically stenotic plaque section **316** is shown to include a severe calcification **354,** and old thrombus **356,** and an eccentric lumen **358.**

Referring now to **Figures 4A-K,** a cross-sectional view of several preferred embodiments of a catheter apparatus of this invention, generally **400**, are shown. Each apparatus **400** includes at least a guide-catheter **402** having housed therein at or near its distal end **404** a contrast agent delivery assembly **406**, a guide wire **408** and a micro-catheter **410** having a first probe **412** slidingly mounted onto the guide wire **408** via an orifice **414** in a center **416** of the probe **412.** Of course, it should be recognized that the components disposed at or near the distal end **404** of the guide-catheter **402** can be inserted into the guide-catheter **402** after guide catheter deployment. Looking at **Figure 4A**, the contrast agent delivery assembly **406** includes a contrast storage element **418**, a pump element **420** and an orifice **422**. Looking at **Figure 4B**, the apparatus **400** of **Figure 4A** further includes a balloon **424** disposed on an outer surface **426** of the guide-catheter **402**. Looking at **Figure 4C**, the apparatus **400** further includes a second probe **428** also slidingly mounted onto the guide wire **408** via an orifice **430** in a center **432** of the probe **428**, where the second probe **428** is designed increase a type and content of data acquired by the apparatus **400.** Looking at **Figure 4D**, the apparatus **400** of **Figure 4C** further includes a balloon **424** disposed on an outer surface 426 of the guide-catheter **402.** Looking at **Figure 4E**, the contrast delivery assembly **406** includes a contrast storage element **418,** a pump element **420**, a conduit **434** and a probe orifice **436.** Looking at **Figure 4F,** the apparatus **400** of **Figure 4E** further includes a balloon **424** disposed on an outer surface **426** of the guide-catheter **402.** Looking at **Figure 4G**, the apparatus **400** of **Figure 4C** wherein the contrast delivery assembly **406** includes a contrast storage element **418**, a pump element **420**, a conduit **434** and a probe orifice **436**. Looking at **Figure 4H**, the apparatus **400** of **Figure 4G** further includes a balloon **424** disposed on an outer surface **426** of the guide-catheter **402.** Looking at **Figure 4I**, the apparatus **400** of **Figure 4C** further including a balloon **424** slidingly mounted onto the guide wire **408** via an orifice **438.** Looking at **Figure 4J**, the apparatus **400** of **Figure 4J**

wherein the contrast delivery assembly **406** includes a contrast storage element **418,** a pump element **420**, a conduit **434** and a probe orifice **436.**

Referring now to **Figures 5A-D,** a cross-sectional view of several other preferred embodiments of a catheter apparatus, generally **500** are shown. Each apparatus **500** includes at least a guide-catheter **502** having housed therein at or near its distal end **504** a contrast agent delivery assembly **506**, a first guide wire **508**, a second guide wire **510**, a first micro-catheter **512** having a first probe **514** slidingly mounted onto the first guide wire **508** via an orifice **516** in a center **518** of the probe **512** and a second micro-catheter **520** having a balloon **522** slidingly mounted onto the second guide wire **510** via an orifice **524** in a center **526** of the balloon **522.** Of course, it should be recognized that the components disposed at or near the distal end **504** of the guide-catheter **502** can be inserted into the guide-catheter **502** after guide catheter deployment. Looking at **Figure 5A**, the contrast agent delivery assembly **506** includes a contrast storage element **528,** a pump element **530** and an orifice **532.** Looking at **Figure 5B**, the apparatus **500** further includes a second probe **534** also slidingly mounted onto the first guide wire **508** via an orifice **536** in a center **538** of the second probe **534**, where the second probe **534** is designed to increase a type and content of data acquired by the apparatus **500.** Looking at **Figure 5C**, the contrast delivery assembly **506** includes a contrast storage element **528,** a pump element **530**, a conduit **540** and a probe orifice **542**. Looking at **Figure 5D**, the apparatus **500** of **Figure 5C** further includes a second probe **534** also slidingly mounted onto the first guide wire **508** via an orifice **536** in a center **538** of the second probe **534**, where the second probe **534** is designed to increase a type and content of data acquired by the apparatus **500.**

Referring now to **Figure 6**, a cross-sectional view of another preferred embodiment of a catheter apparatus, generally **600**, is shown to include a guide-catheter **602** having housed therein at or near its distal end **604** three guide wires **606**, **608**, and **610.** The apparatus **600** also includes a first micro-catheter **612** having a contrast agent delivery system **614** having an orifice **616,** where the first micro-catheter **612** is slidingly mounted onto the first guide wire **606** via an orifice **618** in a center **620** of the system **614**. The apparatus **600** also includes a second micro-catheter **622** having a balloon **624** slidingly mounted onto the second guide wire **606** via an orifice **626** in a center **628** of the balloon **624**. The apparatus **600** also includes a third micro-catheter **630** having a probe **632** slidingly mounted onto the third guide wire **610** via an orifice **634** in a center **636** of the probe **632**. Of course, it should be recognized that the components disposed at or near the distal end **604** of the guide-catheter **602** can be inserted into the guide-catheter **602** after guide catheter deployment.

Referring now to **Figures 7A-C**, a cross-sectional view of several other preferred embodiments of a catheter apparatus, generally **700** are shown. Each apparatus **700** includes at least a guide-catheter **702** having housed therein at or near its distal end **704** a guide wire **706.** Looking at **Figure 7A**, the apparatus **700** also includes a micro-catheter **708** having a first probe **710** slidingly mounted onto the guide wire **706** via an orifice **712** in a center **714** of the first probe **710.** The micro-catheter **708** also includes a contrast agent delivery system **716** having an orifice **718** slidingly mounted onto the guide wire **706** via an orifice **720** in a center **722** of the system **716.** Of course, it should be recognized that the components disposed at or near the distal end **704** of the guide-catheter **702** can be inserted into the guide-catheter **702** after guide catheter deployment. Looking at **Figure 7B**, the apparatus **700** of **Figure 7A** further includes a balloon **724** slidingly mounted onto the guide wire **706** via an orifice **726** in a center **728** of the balloon **724.** Looking at **Figure 7C**, the apparatus **700** of **Figure 7B** further includes a second probe **730** slidingly mounted onto the guide wire **706** via an orifice **732** in a center **734** of the second probe **730,** where the second probe **730** is designed to increase a type and content of data acquired by the apparatus **700.** It should also be recognized that in the catheter apparatuses of **Figures 7A-C,** the order of the micro-catheter components is a matter of design choice and the components can be arranged in any desired order to obtain a desired result.

In all of the catheter embodiments described above, the contrast agent delivery system is designed to inject a contrast agent into a vessel in an animal including a human body so that data collected before and after or before, during and after injection can be compared to determine and quantify areas in which signal enhancement occurs in a transient manner-peaks and fades over time. All of the contrast delivery systems include one or more orifice through which the contrast agents are introduced into the vessel. Although these embodiments all show on board contrast agent delivery systems, it should be recognized that the delivery system can also be external to the guide-catheter or micro-catheter and connected to the orifices by conduits running the entire length of the guide-catheter or the guide-catheter and micro-catheter depending on the location of the orifice. Moreover, the catheters of this invention can include orifices at both the distal end of the guide-catheter and at a location associated with a micro-catheter so that contrast agent can be introduced at two different locations consecutively or simultaneously.

In the catheter apparatuses described above that include a balloon, the balloon and balloon placement are intended to alter blood flow in the vessel after contrast agent introduction altering the time frame for contrast agent diffusion into vessel structures such as the vasa vasorum, plaque or other vessel structures. The alteration of vessel blood flow can be from complete shut down of flow to any intermediate value including the state of non-deployment which would represent the highest flow achievable in the vessel into which the catheter apparatus is deployed. By altering the blood flow before, during or after contrast agent injection, the data collected is and can be used to highlight different anatomical, morphological and/or pathological properties of the vessel being tested and its immediate environment,

where the term immediate environment means all structures surrounding the probe in the catheter that are visible in the data or data images obtained.

## MATERIALS & METHODS

### IVUS System

The inventors used a solid-state phased-array 20 MHz IVUS scanner (available from Volcano Therapeutics Inc.-Invision™) for two cases and a rotating single-crystal 40 MHz IVUS scanner (available from Boston Scientific Inc.-Galaxy™) for another case.

### Microbubbles

The inventors used Optison®, a new ultrasound contrast agent composed of albumin microspheres filled with octafluoropropane gas. While the Optison microbubbles were used in the studies report herein, any other ultrasound microbubble contrast agent can be used as well.

### IVUS Imaging Basics

IVUS imaging has matured over the years. Much research has gone into the segmentation problem in IVUS imaging, specifically for the lumen and media-adventitia interface. Referring now to **Figures 8A&B**, a typical intravascular ultrasound image and the corresponding vessel morphology are shown. The IVUS image shows the following structures: a calcified area composed of calcium, **calcium**, an acoustic shadow region, **Acoustic Shadow**, a plaque region, **Plaque**, a media region, **Media**, an adventitia or surrounding tissue region, **Adventitia** /**Surrounding tissues**, a lumen region, **Lumen**, and a catheter, **Catheter**.

For imaging modalities where acquisition is affected by periodic motion (*e.g.*, due to the heart beat cycles or respiration cycles), there are two general methods for motion compensation: image registration and signal gating. Image registration attempts to match features between two frames in order to explicitly compensate for the relative motion between the two frames. Specifically, after feature matching is performed, one image is warped to roughly correspond to another. Rigid registration maybe performed where one image differs from another only by one or more affine transformations, but due to the elastic nature of the structures imaged by IVUS, this may be an inaccurate assumption. Recently, methods have been developed for elastic registration of IVUS images. The second method of motion compensation, signal-gating, allows well-correlated frames to be extracted from a frame sequence by ignoring those frames that do not correspond to a particular cardiac or respiratory phase. The advantages to this technique are that it can work in real-time and, unlike registration, it does not require digital warping or re-sampling of images in the resulting frame set. In IVUS imaging, pullback studies have been performed using ECG-gated data to gather only phase-correlated frames. Where ECG gating is not available as part of the acquisition hardware, the inventors have developed techniques to derive cardiac phase from the IVUS frame sequence itself by analyzing intensity features over time.

### Imaging Protocol

The method for imaging and detecting vasa vasorum utilizes pre- and post-contrast IVUS image analysis orpre-, during, and post-contrast IVUS image analysis. IVUS signals were continuously collected for a period of time prior to microbubble injection, optionally for a period of time during microbubble injection and for a period after microbubble injection, with the catheter position held fixed. The inventors found specific regions within the images that had increased echogenicity after microbubble injection, these regions are called enhancements. As time progresses, the enhancement fades. In particular, IVUS signals were continuously collected 20 s before and up to 2 min after the injection of microbubble contrast agent with the catheter position held fixed. In studying signals from post-injection frames, we expected to find increased echogenicity in the vasa vasorum or enhancements in specific regions of the IVUS sequence. Referring now to **Figures 9A-C** shows selected IVUS frames acquired pre-injection, during-injection, and post-injection. The observer may focus on the media-adventitia interface to note obvious changes that occur maximally at around the 10-12 o'clock position post-injection and as stated above, as time progresses, the enhancement fades.

The inventors used the following method for acquiring data in a typical stationary-catheter IVUS configuration. This method includes the steps of: (1) acquiring IVUS frames for several minutes of a vessel in which the IVUS catheter is held fixed to obtain non-enhanced data or a pre-injection image sequence, (2) injecting a contrast agent and acquiring during injection data or a during-injection image sequence, which is temporarily washing out due to the echo-opacity of the lumen as it is saturated with the microbubbles, (3) acquiring IVUS frames for several minutes after microbubble injection to obtain data or a post-injection image sequence, which potentially including enhanced areas. Generally, the

method also included a saline inj ection after the microbubble inj ection to flush out any microbubbles in the conduit. However, if the micro-bubble delivery system is on board the micro-catheter, then a flush is not required. For example, after a baseline IVUS image sequence of a site in a coronary artery for which a plaque index is desired, 5 cc of Optison, a clinically available IVUS contrast agent used for non-invasive myocardial perfusion imaging by echocardiography, was injected through the guide-catheter while the IVUS sequence was constantly recorded for 10 s before the injection and for 1 min after the injection. After one min, 5 cc normal saline was injected into through guide-catheter to flush out the remaining microbubbles. The IVUS sequence was recorded for 10 s before and 20 s after saline flush. The post IVUS sequence is then acquired for up to several minutes post saline flush.

## Imaging Vasa Vasorum Density and Plaque Perfusion Analysis

The method relates to vessel micro-morphological imaging such as vasa vasorum detection, plaque detection and the detection of surrounding tissue using an imaging algorithm including two main steps. First, a set **F** of IVUS frames encompassing pre-injection, during-injection, and post-injection periods is formed. The set is then processed to remove cardiac motion to compensate for relative catheter/vessel movement. This is accomplished by analyzing a subset **F'** $\subset$ **F** of the frame sequences, where each frame in **F'** is associated with approximately a same point in a cardiac phase. Second, enhancement detection is performed on these sets of phase-correlated frames, using difference-imaging and statistical techniques described in more detail herein.

## Motion Compensation

In stationary-catheter IVUS data acquisition, a constant relative catheter/vessel position is impossible to achieve in practice due to periodic heart and respiratory movements during data acquisition, where the movements change the relative position during each heart and/or respiratory cycle. Thus, during data acquisition, this relative motion between the catheter and the vessel produces image sequences in which each frame deviates from its predecessor depicting data from a slightly different anatomical location in the vessel. These deviations make image analysis of a specific anatomic region-of-interest (ROI) difficult, because data associated with a specific frame region in the sequence of acquired frames may not correspond to a same physical region of the vessel being diagnosed. From a mathematical perspective, this periodic motion gives rise to certain degrees of freedom: (a) catheter/vessel rotation - the movement of the catheter toward and away from the vessel wall (X-Y translation), and (b) the incidental movement of the catheter along the length of the vessel (Z-translation). It should be noted that Z-translations can cause radical changes in the appearance of individual frames in a IVUS sequence of acquired frames. In particular, some features of the vessel under examination may be only intermittently visible as they move into and out of a plane associated with the IVUS acquisition. The IVUS sequences were performed without associated ECG data. Consequently, the present method allows cardiac phase information to be derived from the IVUS sequence itself. Specifically, intensity changes in the IVUS sequence are tracked over a particular fixed ROI of the IVUS frame. These intensity changes provide a 1-D signal which, when filtered for noise, exhibits peaks and troughs that are associated with specific (though arbitrary) points in cardiac cycles. The steps performed for cardiac phase extraction include ROI selection, intensity signal generation, filtering and signal reconstruction.

## ROI Selection

Selecting aregion-of-interest (ROI) involves identifying a particular area of a given frame or image and making the identified area a fixed ROI for analyzing the IVUS frame sequence. Changes in intensities that occur in this RIO over time are monitored. Data from the entire frame is not analyzed as a whole for two reasons. First, the lumen/catheter region produces little useful signal and, the signal from the adventitial region has a very low signal-to-noise ratio (SNR). Second, the method is more effective when some features enter and exit the ROI periodically due to cardiac motion; this cannot occur if the entire frame is used. Consequently, the preferred ROIs for use in this invention are regions between a luminal border and a adventitia (plaque+media) of the artery. In these regions, much of the coherent signal is located. The ROI need not be a perfect segmentation of this region, however. In fact, an average IVUS frame can be constructed from image data over a particular time range (*e.g.*, one or two cardiac periods). The average IVUS frame thus represents a mask which, on average, contains the entire ROI as shown in **Figures 10**.

## Intensity Signal Generation

To produce the intensity signal, two techniques are used: an average intensity technique and an inter-frame difference technique. The average intensity $g$ at frame $\boldsymbol{I}$ over the selected ROI is given by

$$g_i = \frac{1}{n} \sum_{(x,y)\in ROI} F_i(x,y)$$

where ***F*** is a given IVUS frame. The inter-frame difference ***d*** between the current frame ***I*** and the previous frame ***i-1*** is given by

$$d_i = \frac{1}{n} \sum_{(x,y)\in ROI} \left| F_i(x,y) - F_{i-1}(x,y) \right|$$

In both cases, ***n*** is the number of pixels corresponding to the ROI in the frame. In the present method , these techniques are used interchangeably; the only apparent difference between these two intensity signal generation methods is a phase shift between the resulting graphs. Also, in many cases, there may be two peaks or troughs associated with each cardiac period due to the same relative catheter/vessel position is attained twice during each cycle. Consequently, "phase-correlated" frames also include frames correlated by common catheter/vessel orientation.

**Filtering & Signal Reconstruction**

Due to the many spurious peaks and troughs in the signals $g_i$ and $d_i$ produced by these methods as shown in **Figure 11A**, it is necessary to apply a filter to make finding these points a simple matter of locating local maxima and minima. The preferred filter for use in the method of this invention is a Butterworth bandpass filter given by the following equation:

$$H(\omega) = \frac{1}{\sqrt{1 + \left[ \frac{2(\omega - \omega_c)}{\Delta\omega} \right]^{2n}}}$$

where $\Delta\omega=0.6(\omega_c)$, $n=4$, and $\omega_c$ is centered at a dominant cardiac frequency. $\Delta\omega$ and ***n*** denote a width and an order of the filter, respectively. In stationary-catheter IVUS studies, the method is robust with regard to automatic cardiac phase extraction, and signals produced using this method generate an obvious peak in the frequency domain as shown in **Figure 11B**. This makes automatic location of the dominant frequency feasible. In this case, other low-frequency peaks are ignored. Specifically, in addition to a prominent DC component due to the mean intensity or the inter-frame difference, there will also be other associated low-frequency components. These other low-frequency components are due to a contrast injection during acquisition, which causes a varying mean grey-level to occur in the ROI over time (*i.e.*, the washout during inj ection and the enhancement effect post-injection). Once the frequency-domain signal is filtered as shown in **Figure 11C**, it is converted back into a time domain signal using an inverse Fast Fourier Transform as shown in **Figure 11D.** The set of frames associated with, *e.g.*, the peaks in the graph, are considered or defined to be correlated for purposes of this method. As a side benefit, the data can also be used to determine an average heart rate in beats per minute during the sequencing diagnosis. The signal shown in **Figure 11D**, for example, has an average wavelength of 10.6 frames in a sequence sampled at 10 frames/s, giving a heart rate to ~64 beats/min.

**Enhancement Detection**

The goal of enhancement detection is to localize and quantify subtle intensity changes in the IVUS image sequence due to a portion of the vasa vasorum present in a field of view of the IVUS catheter. These localized intensity changes are due to a perfusion of contrast agent micro-bubbles into the vasa vasorum during the micro-bubble injection step.

Matching points between adj acent frames in the acquired sequence during a cardiac cycle are referred to as

"phase-correlated frames" or simply as "correlated frames." These correlated frames are extracted from the IVUS frame sequence using the motion-compensation method discussed previously, which is the preferred motion-compensation technique. Other methods can also be used to accomplish the same task for example ECG-gating.

For enhancement detection to be reliable, it must be invariant to the presence of speckle noise in the IVUS image. Thus, the enhancement detection method of this invention includes two steps: (a) noise reduction to attenuate speckle noise and (b) difference imaging to detect the enhancement itself. For quantification of the enhancement, statistics computed from the difference images are used.

## Noise Reduction

An inherent characteristic of coherent imaging, including ultrasound imaging, is the presence of speckle noise. Speckle is a random and deterministic interference pattern in a signal formed with coherent excitation of a medium and is caused by the presence of sub-resolution scatterers. The local intensity of a speckle pattern reflects a local echogenicity of an underlying scatterers and depends on a scatterer density and coherence. Thus, speckle has a multiplicative effect on an amplitude of a radio-frequency (RF) signal, which is much more prominent than any additive measurement error on the RF signal itself.

In the method, noise reduction is accomplished by temporally averaging frames. Temporal averaging is commonly used in ultrasonic imaging due to an inherent difficulty in separating speckle noise from subtle coherent events and due to a poor quality of individual frames in most ultrasonic modalities. The actual enhancement effect itself differs little in appearance from a typical speckle noise pattern due to the small scale of the vasa vasorum, so the various spatial filtering techniques that have been developed for ultrasonic imaging may eliminate the very features that are being sought.

## Differential Imaging

Difference imaging is a technique used ubiquitously in the fields of remote sensing and astronomy as a method for detecting temporal change. The enhancement effect sought to be detected in this invention is a localized, coherent grayscale intensity increase apparent in the IVUS sequence after an injection of an amount of a micro-bubble ultrasonic contrast agent. The enhancement is due to a perfusion of micro-bubble contrast agent into the vasa vasorum, as previously discussed. In appearance, the enhancement differs little from that of ultrasound speckle noise in most cases. The main visual difference between the enhancement and speckle noise is that the enhancement is temporally more coherent, though it is by no means a permanent feature of a post-injection IVUS frame sequence; enhancement may disappear from some areas in a matter of seconds following the passing of the micro-bubbles.

Difference imaging typically involves a "before" image and an "after" image. For enhancement detection, our before image $\boldsymbol{I_b}$ is a frame derived from a set of phase-correlated pre-injection frames by temporal averaging for noise reduction, as discussed previously. That is, if a set of $\boldsymbol{n}$ phase-correlated pre-injection frames $F_n \equiv \{F_1 \ldots F_n\}$, the average $\overline{F}$ is defined by

$$\overline{F}(x,y) = \frac{1}{n}\sum_{i=1}^{n} F_i(x,y)$$

where the number of frames $\boldsymbol{n}$ that are averaged to produce this image (*i.e.*, the temporal window) can be fairly large depending on the amount of data available, since we are interested only in highly temporally-coherent features of the IVUS sequence for this baseline image. The after image $\boldsymbol{I_a}$ is either (1) a single post-injection frame, or (2) an average of correlated post-injection frames. A single frame is useful if producing a movie sequence (to be discussed herein), while a temporal average of a post-injection set of images to produce $\boldsymbol{I_a}$ will suppress speckle noise along with temporally incoherent enhanced regions. Longer-period temporal averaging will suppress more noise and bring out only highly temporally-coherent enhancement. [0146] Once $\boldsymbol{I_B}$ and $\boldsymbol{I_a}$ images are available, a grey-level difference image is produced using pixel-by-pixel subtraction, $\boldsymbol{I_d}(\underline{x},y) = \boldsymbol{I_a}(x,y)-\boldsymbol{I_b}(x,y)$, with negative values thresholded set to zero. This difference image will show some areas of true enhancement plus low-valued areas of false enhancement due to speckle noise (depending on the amount of noise reduction performed previously). Thresholding may be applied to reduce the noisy appearance of the resulting difference image, but this must be done carefully to avoid suppressing relevant enhancement data while attenuating the effect of low-level speckle noise. In this context, the present method employs an automatic thresholding technique utilized previously for remote-sensing change detection applications. A grey-level threshold is determined for the difference image under the assumption that it is a Bayesian mixture of probabilities given by

$$p(X) = p(X / \upsilon_n)P(\upsilon_n) + p(X / \upsilon_c)P(\upsilon_c)$$

where $p(\boldsymbol{X})$ is an overall probability density function of the difference image, $\boldsymbol{p}(\boldsymbol{X}/\upsilon_n)$ and $\boldsymbol{p}(\boldsymbol{X}/\upsilon_c)$ are probability densities of distributions of non-enhanced (***n***) and enhanced (***c***) pixels, respectively, and $\boldsymbol{P}(\upsilon_n)$ and $\boldsymbol{P}(\upsilon_n)$ are *a priori* probabilities of these classes of pixels. Following this global thresholding, a Markov modeling technique is then applied to the function to account for spatial relationships, *i.e.*, to reduce spot noise while refining edges of enhanced regions. Note that for purposes of measuring probabilities and enhancement levels, only the pixels in the ROI are taken into consideration; typically, the intima-medial region is the preferred ROI, the ROI where we expect to find the vasa vasorum relevant data. However, this ROI need not be a perfect segmentation of the intima-medial region, such an ROI can be used to simultaneously for cardiac cycle determination.

Difference-image sequences may be generated to show the changes in microbubble perfusion in the vasa vasorum over time. In this case, the "before" image $\boldsymbol{I_b}$ is kept constant to the pre-injection average, while the "after" images $\boldsymbol{I_a}$ are a sequence of either individual phase-correlated post-injection frames or a running temporal average of the same. In general, if a sequence of ***n*** correlated post-injection frames $\boldsymbol{Cn} \equiv \{\boldsymbol{C_1} \ldots \boldsymbol{C_n}\}$ is available and an ***m***-frame running average is used (where *m* is odd), a ***k***-frame animation may be produced, where $\boldsymbol{k} = \boldsymbol{n} - \boldsymbol{m} + 1$ and the difference image for frame $\boldsymbol{F_i}$ ($\boldsymbol{i} \in [1, \boldsymbol{k}]$) is defined as

$$F_i = \left( \frac{1}{m} \sum_{j=1}^{m} C_{i+j-1} \right) - I_b$$

where the operations are performed on a pixel-by-pixel basis.

## RESULTS

### Data

After initial experiments, we conducted microbubble contrast-enhanced IVUS imaging in 7 patients with coronary artery disease. Due to space limitations, data collected from 3 cases only are presented.

IVUS images were acquired at resolutions of 384x384, 480x480, and 578x578 pixels, respectively, for the 3 cases. Images were acquired using the DICOM standard at either 10 or 30 frames/s. The analyses were run on a Pentium-IV based 2 GHz PC. The IVUS baseline images were typically recorded for a period of 30 s to 3 min prior to contrast agent injection, after which contrast agent was injected and a contrast-enhanced sequence was recorded for a similar period of time.

### Case 1

The catheters described above were inserted into the case 1 patient and the probe situated in a location of the coronary artery to be imaged and a 1.8-minute sequence including 1,073 frames sampled at 10 frames/s was acquired. The sequence included before injection, during injection and post injection frames. After microbubble contrast agent injection, the microbubbles first appear in the lumen in frame 404, complete washout due to lumen echo-opacity occurred between frames 452-490, and the microbubbles reach a minimum in lumen area around frame 530. The catheter was removed beginning at frame 1,044. For this study, we have approximately 400 pre-injection frames and 500 post-injection frames. Representative frames from this set are shown in **Figures 12A-C**. A manual segmentation of a frame from this set makes up the static image described in **Figures 8A&B** described above.

### Case 2

The catheters described above were inserted into the case 2 patient and the probe situated in a location of the coronary artery to be imaged and a 15-minute sequence including 9,000 frames sampled at 10 frames/s was acquired. The sequence included before injection, during injection and post injection frames. After microbubble contrast agent injection, the microbubbles first appears in the lumen in frame 1,797 and complete washout occurred at frame 2,609. For this study, we acquired approximately 1,700 pre-injection frames and 6,000 post-injection frames. Representative

frames from this set are shown in **Figures 12D-F.**

## Case 3

The catheters described above were inserted into the case 3 patient and the probe situated in a location of the coronary artery to be imaged and a 1.05-minute sequence including two separate subsequences. The first subsequence includes 792 pre-injection frames, while the second subsequence includes 1,096 post-injection frames. The frames in each subsequence were acquired at 30 frames/s. The sequence included before injection, during injection and post injection frames. After microbubble contrast agent injection, the microbubbles first appears in the lumen in the latter set at frame 82. Unless otherwise noted, frame numbers associated with this case refer to frames in the post-injection subsequence. Representative frames from this set are shown in **Figures 12G-I.**

## Visualization and Quantification of Results

Thresholded difference images refer to difference images that have been automatically thresholded, yet retain their original grey levels for all values over the threshold. Binary-thresholded difference images refer to images that have been similarly thresholded, but no longer contain their original grey levels and hence, ideally, illustrate only points showing true enhancements. Note that because the enhancement statistics are computed only over the selected ROI, the enhancement effects illustrated here are only valid in the intimo-medial area of the IVUS image, i.e., the area as shown highlighted in **Figure 15.** The adventitial regions have been included in the figures for completeness, but frequently contain spurious responses, especially due to acoustic shadowing effects as example of which was shown in **Figure 8A**.

The method quantifies an amount of enhancement brought about by the contrast-agent injection as follows. Enhancement graphs, as shown for example in **Figures 16A&B**, are produced which measure the average enhancement per enhanced pixel (AEPEP) in the ROI over time. The average is determined as follows. Let $\mathbf{P}_i = \{P_1, ..., P_n\}$ be a set of intensities of enhanced pixels in the ROI of the difference image (where, if $T_i$ is the enhancement threshold, then $\forall_{p \in P_i}, p > T_i$), then an AEPEP value $\varepsilon_i$ for frame $I$ is given by

$$\varepsilon_i = \frac{1}{n_i} \sum_{p \in \mathbf{P}_i} p$$

A useful feature of the AEPEP method is that it remains fairly constant in spite of temporal changes due to perfusion; that is, the average is robust to apparent changes in the area of the enhanced regions. This property provides a stable indicator of overall enhancement for comparison to the pre-injection sequence. Other metrics such as a total enhanced area (in pixels) or a sum of intensities of enhanced areas are also useful metrics. Metrics utilizing the average or summed intensities over the *entire* ROI were found to be too noisy and less reliable measures of enhancement as compared to the AEPEP method and as shown in **Figure 11A**. These latter metrics are less reliable mainly because the area of the enhancement effect are extremely small compared to the overall area of the ROI. Smoothing is typically performed on the resulting graphs to separate the long-term effects of enhancements from effects of ultrasound noise.

Where results are reported as percentages (%), the percentages refer to the AEPEP measure divided by the maximum grey-level difference (255). This measure is useful for raw difference images as shown in

## ANALYSIS

## Case 1

**Figure 13** illustrates a raw difference image showing marked enhancement in the plaque region, particularly in the region at about 6 o'clock approximately midway between the two maj or calcium deposits evident at about 10 o'clock and 12 o'clock. Representative thresholded difference images during and post injection are shown in **Figure 14A&B** and representative binary-thresholded difference images during and post injection are shown in **Figure 15A&B,** which illustrate changes in enhancement over time due to perfusion and diminution. The enhanced regions were seen to reduce in area and strength over the length of the dataset, but remain coherent. Enhancement was quantified over time for this case as shown in **Figures 16A&B** and as annotated in **Figures 17A&B** and separately for both the intimo-medial/plaque and adventitial regions.

**Case 2**

Representative thresholded difference images during and post injection are shown in **Figure 18A&B** and representative binary-thresholded difference images during and post injection are shown in **Figure 19A&B**, which illustrate changes in enhancement over time due to perfusion and diminution. The quality of the acquired data for this case was found to be the poorest of the three cases, especially with respect to ultrasound noise and alignment of image features using the automated cardiac phase detection method. Low-level enhancement visible along the arc between about 2 o'clock and about 4 o'clock is mainly due to slight misregistration of the bright calcium plaque between the before and after image sets. Most of the high-level coherent enhancement can be seen at about 3 o'clock. Enhancement was quantified over time for this case as shown in **Figures 20A&B** and as annotated in **Figures 21A&B** and separately for both the intimo-medial/plaque and adventitial regions.

**Case 3**

Representative thresholded difference images during and post injection are shown in **Figure 22A&B** and representative binary-thresholded difference images during and post injection are shown in **Figure 23A&B**, which illustrate changes in enhancement over time due to perfusion and diminution. The most coherent enhancements seen in this dataset were located at between about 9 o'clock and about 10 o'clock of **Figure 22B**. They are invisible on the during-injection frame (which mainly highlights remnant contrast agent in the lumen and shadowing effects in the adventitia), indicating the contrast agent took some time to perfuse into the vasa vasorum. Enhancement was quantified over time for this case as shown in **Figure 24** and as annotated in **Figures 25A&B** and separately for both the intimo-medial/plaque and adventitial regions.

**Endoluminal Enhancement and Intraluminal Flow**

The motion of the microbubbles in the annular region between the IVUS catheter and the lumen has a critical effect on the generated signal. Two important factors in this regard are the boundary layers near the walls and the unsteady pulsatility of the flow that enhances the mixing between the injected fluid and blood. The passage of the main bubble cloud past the IVUS imaging sensor produces a signal blackout. For microbubbles to be perfused into the vasa vasorum for subsequent imaging, they must pass through the endothelial wall boundary layers, where the speed of the injected fluid becomes very slow. Thus, the boundary layers can hold a residue of microbubbles long after the bulk of the injected microbubble cloud has washed downstream. In addition, there is evidence of selective adherence of microbubbles to leukocytes and regions of endothelial inflammation. The result of these last two effects can be an enhancement of the endoluminal surface in the subsequent IVUS images. By moving the delivery orifice for the microbubbles past the ultrasound probe or other probe, the blackout period can be minimized or even eliminated. Thus, by changing the point of injection of the contrast agent, the washout period can be changed as well as the rate of perfusion of the microbubbles into ROIs, whethertheROI is associated with a coronary artery, a peripheral artery, a malignancy, or other tissue site that is fed by micro-vessels.

**DISCUSSION**

We have presented a method for imaging vasa vasorum density and perfusion of atherosclerotic plaques using contrast-enhanced intravascular ultrasound imaging. Our findings show for the first time that microbubble-induced ultrasound signal enhancement in the coronary artery wall can be imaged by high frequency IVUS catheters, where high frequency means a frequency between about 20 and about 40 MHz.

We found in a previous study in dogs that the relative contrast enhancement in the coronary artery itself (*i.e.*, the area under the time-intensity curve) is proportional to the amount of agent injected and inversely related to blood flow at the injection site because of dilution. The enhancement in tissue is thus a function of blood/tissue volume as well as the injection volume and dilution at the injection site. In assessing myocardial perfusion, normally-perfused regions are commonly compared to suspect regions to compensate for the variability in injection volumes and rates and for dilution by blood. Thus, the relative contrast enhancement between regions in tissues is a measure of relative vascular volume or of vasa vasorum density in blood vessel walls, assuming that the agent remains in the vascular space and does not become trapped. The extended enhancement seen in some regions of high vascularity, and presumably high perfusion, suggests either leakage into the surrounding tissue or trapping of microbubbles at the capillary level.

We have found scattered and spotted distribution of microbubbles in the wall. The spotted enhancement seen in our post-contrast images (versus diffused and widespread background enhancement) suggests that due to limited spatial resolution of IVUS, we are likely to image clouds of microbubbles instead of single microbubbles. Such clouds are likely to be imaged in larger branches of vasa vasorum. A comparison between our images and the micro CT and

pathology images of vasa vasorum reported in the literature showed conceptual agreement. We also were able to monitor the change in IVUS signal intensity over time as a measure of plaque perfusion. Since perfusion inside the plaque is via microcapillaries, the perfusion and clearance rate of microbubbles is slower than the clearance rate of intraluminal (intracoronary) microbubbles. This allows extended monitoring of vasa vasorum perfusion. In certain plaques, presumably those with extensive inflammation, most vasa vasorum are fragile and leaking. Such vasa vasorum may exhibit leakage of microbubbles, and create an imaging trace namely "blush sign" as suggested previously. In our study, we have found an indication of IVUS blush sign in one case (see the residual rise in contrast enhancement as shown in the last frames of **Figures 16A&B**.

The results presented here are superior using an automated cardiac phase determination than has been achieved previously demonstrating the superiority of the present methodology. This is due to the fact that in our stationary-catheter study, coherent changes in image features are almost entirely due to cardiac motion, rather than to a combination of cardiac motion and changing vessel features as the catheter is pulled back. While stationary-catheter placement is preferred, the catheter can be also be moved at a controlled rate of speed to allow for imaging along a given arterial section provided that the motion is controlled in such as way as to allow pre and post injection images to be obtained at each site along the section. This latter method can best be performed by insuring the microbubble injection occurs upstream of the probe so that no washout period occurs. Generally, the motion is a set of moves and holds so that sufficient images can be acquired at each site along the section.

While other have used intravascular molecular targeting techniques for ultrasound vasa vasorum imaging, the present invention produces adequate results without going to such extraordinary measures, because microbubbles are small enough having a diameter between about 1 and about 3 microns and can easily enter into vasa vasorum and circulate throughout the total microcapillary network of plaque.

The SNR of our IVUS vulnerable plaque detection method can be improved by utilizing the observed tendency of albumin microbubbles to adhere to activated leukocytes and enhanced using specially designed microbubbles such as the recently-reported use of microbubbles containing a naturally occurring leukocyte material (Sialyl Lewis X) that binds to adhesion molecules expressed by inflamed endothelial cells. The combination of endothelial surface microbubble adhesion along with indications of vasa vasorum angiogenesis would result in a much stronger marker for vulnerable plaque. In addition, the tendency ofmicrobubbles to adhere to inflamed endothelial cells causes a reduction of flow into the vasa vasorum, and further enhances the imaging of vulnerable plaques by IVUS of this invention. The speed of microbubble perfusion into the vasa vasorum would be decreased, while the microbubble residence time within the vasa vasorum would be increased. As stated above, balloons can also be used to augment the flow of microbubbles into areas of interest. The balloon can be positioned various locations to force diffusion of the microbubbles into desired regions in the target site. It should also be recognized that high-speed acquisition of the raw RF signal would allow the method to detect and quantify more subtle changes in the intensity of the backscattered ultrasound signal inside the plaque and adventitia.

All references cited herein are incorporated by reference. While this invention has been described fully and completely, it should be understood that, within the scope of the appended claims, the invention may be practiced otherwise than as specifically described. Although the invention has been disclosed with reference to its preferred embodiments, from reading this description those of skill in the art may appreciate changes and modification that may be made which do not depart from the scope of the invention as described above and claimed hereafter.

## Claims

1. A catheter apparatus (400, 500, 600, 700) comprising:

    a guide-catheter (402) adapted to be inserted into a peripheral vessel and then positioned in a target vessel;
    a contrast agent delivery system (406) designed to inject an amount of contrast agent into the vessel, the contrast agent delivery system comprising a contrast storage element (418) for storage of the contrast agent and a pump element (420) coupled to the contrast storage element (418); **characterised by**:

        a data collection and data analyzer unit for acquiring pre-injection data prior to injection of the contrast agent into a site and post-injection data after injection of the contrast agent into a site and for correlating the pre-injection data and the post-injection data with a cardiac phase to obtain phase-correlated pre-injection data and phase-correlated post-injection data.

2. The apparatus of claim 1, further comprising:

    at least one guide-wire (408) adapted to be extended from a distal end (404) of the guide-catheter (402) into

the vessel; and
at least one micro-catheter (410) having a central orifice (414) and
adapted to slide down the guide wire (408) to a desired location in the vessel.

**3.** The apparatus of claims 1 or 2, further comprising:

a balloon (424) adapted to augment a flow of blood in the vessel.

**4.** The apparatus of claims 1 or 2, wherein the micro-catheter (410) includes a probe (412).

**5.** The apparatus of claims 1 or 2, wherein the micro-catheter (410) includes a plurality of probes (412, 428).

**6.** The apparatus of claims 2, 3, 4, or 5, wherein the contrast agent delivery system (614) forms a part of the micro-catheter (612).

**7.** The apparatus of claim 6, wherein the contrast agent delivery system (716) is distal of the probe or probes (710, 730).

**8.** The apparatus of claims 3, 4, 5, 6, or 7, wherein the balloon (724) is distal of the probe (710, 730).

**9.** The apparatus of claims 3 or 4, wherein the probe (412; 428) is selected from the group consisting of an ultrasound probe, a variable frequency ultrasound probe, a magnetic probe, a photonic probe, a near Infrared probe, a terahertz probe, microwave probe and combinations thereof.

**10.** The apparatus of claims 1 or 2, wherein the apparatus comprises a contrast agent and the contrast agent is selected from the group consisting of microbubbles, magnetically active microbubbles, magnetically active nanoparticles, in the near infrared range detectable microbubbles, in the near infrared range detectable nanoparticles, optically visible microbubbles, optically visible nanoparticles, in the terahertz range detectable microbubbles, in the terahertz range detectable nanoparticles, in the microwave range detectable microbubbles, in the microwave range detectable nanoparticles and red blood, cells including:
magnetically active nanoparticles, in the near infrared range detectable nanoparticles, optically visible nanoparticles, in the terahertz range detectable nanoparticles, in the range detectable nanoparticles, and mixtures thereof,
and mixtures or combinations thereof.

## Patentansprüche

**1.** Kathetervorrichtung (400, 500, 600, 700), welche aufweist:

einen Führungskatheter (402), der ausgebildet ist, in ein peripheres Gefäß eingeführt und dann in einem Zielgefäß positioniert zu werden;
ein Kontrastmittel-Zuführungssystem (406), das ausgebildet ist zum Injizieren einer Menge von Kontrastmittel in das Gefäß, welches Kontrastmittel-Zuführungssystem ein Kontrastspeicherelement (418) zum Speichern des Kontrastmittels und ein Pumpenelement (420), das mit dem Kontrastspeicherelement (418) gekoppelt ist, aufweist; **gekennzeichnet durch**:

eine Datensammel- und Datenanalyseeinheit zum Erwerben von Vorinjektionsdaten vor der Injektion des Kontrastmittels in eine Stelle und von Nachinjektionsdaten nach der Injektion des Kontrastmittels in eine Stelle und zum Korrelieren der Vorinjektionsdaten und der Nachinjektionsdaten mit einer Herzphase, um phasenkorrelierte Vorinjektionsdaten und phasenkorrelierte Nachinjektionsdaten zu erhalten.

**2.** Vorrichtung nach Anspruch 1, weiterhin aufweisend:

zumindest einen Führungsdraht (408), der ausgebildet ist, sich von einem distalen Ende (404) des Führungskatheters (402) in das Gefäß zu erstrecken; und
zumindest einen Mikrokatheter (410) mit einer zentralen Öffnung (414), der ausgebildet ist, den Führungsdraht (408) bis zu einer gewünschten Stelle in dem Gefäß herunterzugleiten.

**3.** Vorrichtung nach Anspruch 1 oder 2, weiterhin aufweisend:

einen Ballon (424), der ausgebildet ist zum Vergrößern der Blutströmung in dem Gefäß.

**4.** Vorrichtung nach Anspruch 1 oder 2, bei der der Mikrokatheter (410) eine Sonde (412) enthält.

**5.** Vorrichtung nach Anspruch 1 oder 2, bei der der Mikrokatheter (410) mehrere Sonden (412, 428) enthält.

**6.** Vorrichtung nach den Ansprüchen 2, 3, 4 oder 5, bei der das Kontrastmittel-Zuführungssystem (614) einen Teil des Mikrokatheters (612) bildet.

**7.** Vorrichtung nach Anspruch 6, bei der das Kontrastmittel-Zuführungssystem (716) distal von der Sonde oder den Sonden (710, 730) ist.

**8.** Vorrichtung nach den Ansprüchen 3, 4, 5, 6 oder 7, bei der der Ballon (724) distal von der Sonde (710, 730) ist.

**9.** Vorrichtung nach Anspruch 3 oder 4, bei der die Sonde (412; 428) ausgewählt ist aus der Gruppe bestehend aus einer Ultraschallsonde, einer Ultraschallsonde mit variabler Frequenz, einer Magnetsonde, einer photonischen Sonde, einer Nahinfrarotsonde, einer Terahertzsonde, einer Mikrowellensonde und Kombinationen hiervon.

**10.** Vorrichtung nach Anspruch 1 oder 2, bei der die Vorrichtung ein Kontrastmittel aufweist und das Kontrastmittel ausgewählt ist aus der Gruppe bestehend aus Mikroblasen, magnetisch aktiven Mikroblasen, magnetisch aktiven Nanopartikeln, im Nahinfrarotbereich erfassbaren Mikroblasen, in dem nahen Infrarotbereich erfassbaren Nanopartikeln, optisch sichtbaren Mikroblasen, optisch sichtbaren Nanopartikeln, im Terahertzbereich erfassbaren Mikroblasen, in dem Terahertzbereich erfassbaren Nanopartikeln, in dem Mikrowellenbereich erfassbaren Mikroblasen, in dem Mikrowellenbereich erfassbaren Nanopartikeln und roten Blutzellen, enthaltend:

magnetisch aktive Nanopartikel, in dem nahen Infrarotbereich erfassbare Nanopartikel, optisch sichtbare Nanopartikel, in dem Terahertzbereich erfassbare Nanopartikel, in dem Mikrowellenbereich erfassbare Nanopartikel und Mischungen hiervon
und Mischungen oder Kombinationen hiervon.

## Revendications

**1.** Appareil de cathéter (400, 500, 600, 700) comprenant :

un guide de cathéter (402) adapté pour être inséré à l'intérieur d'un vaisseau périphérique puis positionné dans un vaisseau cible ;
un système de délivrance d'agent de contraste (406) conçu pour injecter une quantité d'agent de contraste à l'intérieur du vaisseau, le système de délivrance d'agent de contraste comprenant un élément de stockage de contraste (418) pour le stockage de l'agent de contraste et un élément de pompe (420) couplé à l'élément de stockage de contraste (418), **caractérisé par**:

une unité de collecte de données et d'analyseur de données pour acquérir des données de pré-injection avant l'injection de l'agent de contraste à l'intérieur d'un site et des données de post-injection après l'injection de l'agent de contraste à l'intérieur d'un site et pour corréler les données de pré-injection et les données de post-injection avec une phase cardiaque afin d'obtenir des données de pré-injection corrélées en terme de phase et des données de post-injection corrélées en terme de phase.

**2.** Appareil selon la revendication 1, comprenant en outre :

au moins un guide de fil (408) adapté pour être étendu depuis une extrémité distale (404) du guide de cathéter (402) à l'intérieur du vaisseau ; et
au moins un micro-cathéter (410) comportant un orifice central (414) et adapté pour coulisser en bas du guide de fil (408) jusqu'à un emplacement souhaité dans le vaisseau.

**3.** Appareil selon la revendication 1 ou 2, comprenant en outre :

un ballon (424) adapté pour augmenter une circulation sanguine dans le vaisseau.

**4.** Appareil selon la revendication 1 ou 2, dans lequel le micro-cathéter (410) inclut une sonde (412).

**5.** Appareil selon la revendication 1 ou 2, dans lequel le micro-cathéter (410) inclut une pluralité de sondes (412, 428).

**6.** Appareil selon la revendication 2, 3, 4 ou 5, dans lequel le système de délivrance d'agent de contraste (614) forme une partie du micro-cathéter (612).

**7.** Appareil selon la revendication 6, dans lequel le système de délivrance d'agent de contraste (716) est distal par rapport à la sonde ou aux sondes (710, 730).

**8.** Appareil selon la revendication 3, 4, 5, 6 ou 7, dans lequel le ballon (724) est distal par rapport à la sonde (710, 730).

**9.** Appareil selon la revendication 3 ou 4, dans lequel la sonde (412 ; 428) est choisie parmi le groupe constitué par une sonde à ultrasons, une sonde à ultrasons de fréquence variable, une sonde magnétique, une sonde photonique, une sonde à infrarouges proches, une sonde à térahertz, une sonde à micro-ondes et des combinaisons afférentes.

**10.** Appareil selon la revendication 1 ou 2, dans lequel l'appareil comprend un agent de contraste et l'agent de contraste est choisi parmi le groupe constitué par des microbulles, des microbulles magnétiquement actives, des nanoparticules magnétiquement actives, des microbulles détectables dans la plage des infrarouges proches, des nanoparticules détectables dans la plage des infrarouges proches, des microbulles optiquement visibles, des nanoparticules optiquement visibles, des microbulles détectables dans la plage des térahertz, des nanoparticules détectables dans la plage des térahertz, des microbulles détectables dans la plage des micro-ondes, des nanoparticules détectables dans la plage des micro-ondes et des globules rouges incluant:

des nanoparticules magnétiquement actives, des nanoparticules détectables dans la plage des infrarouges proches, des nanoparticules optiquement visibles, des nanoparticules détectables dans la plage des térahertz, des nanoparticules détectables dans la plage des micro-ondes et des mélanges de celles-ci,
et des mélanges ou combinaisons afférentes.

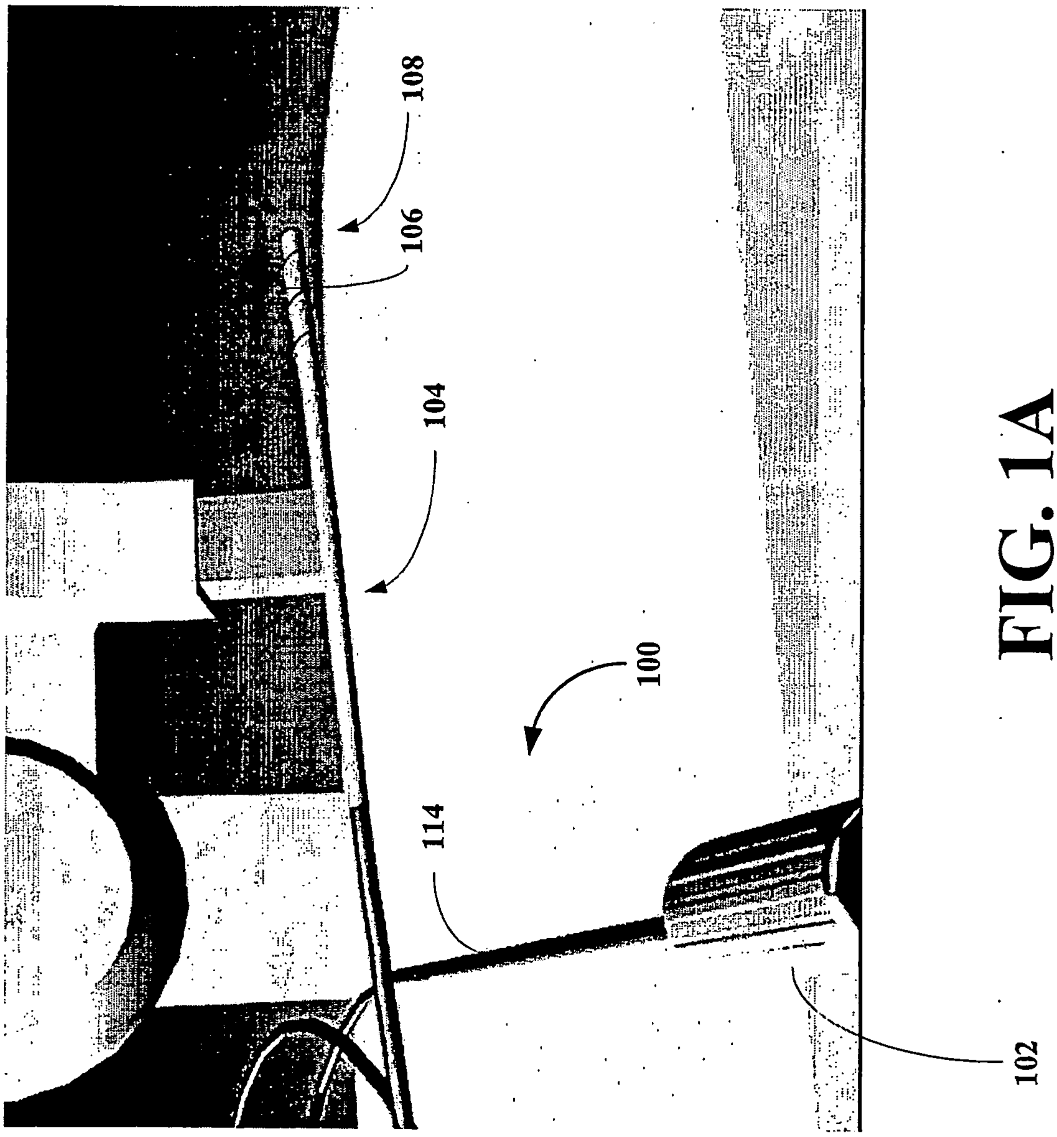


FIG. 1A

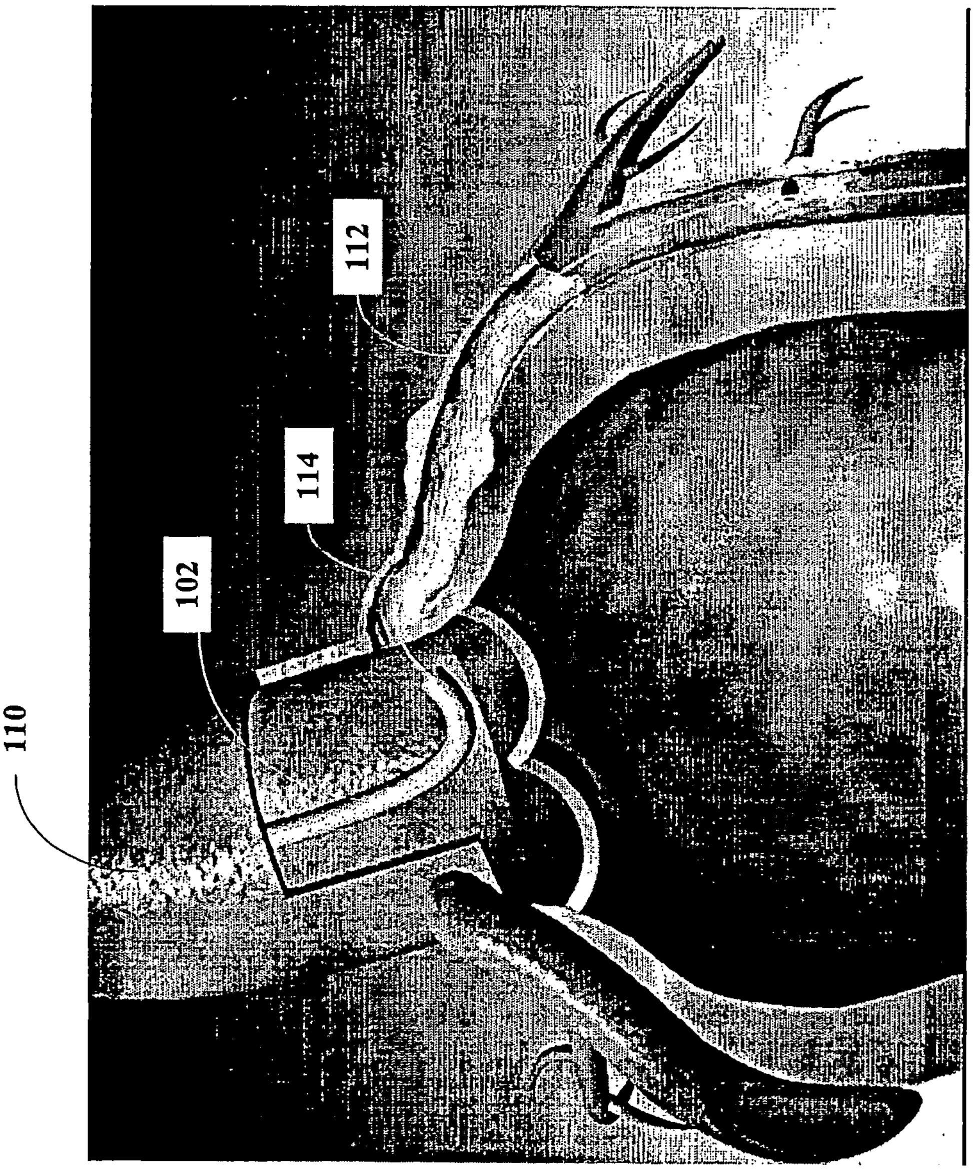


FIG. 1B

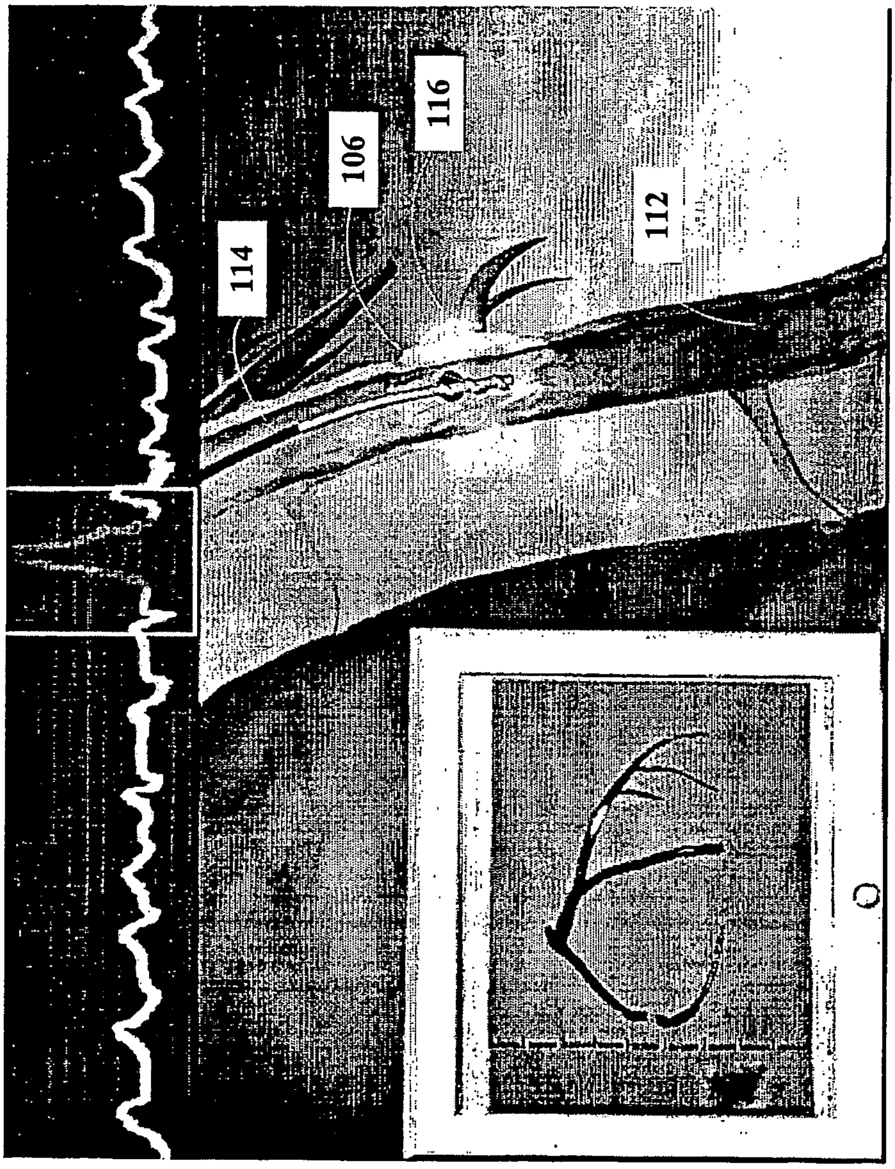


FIG. 1C

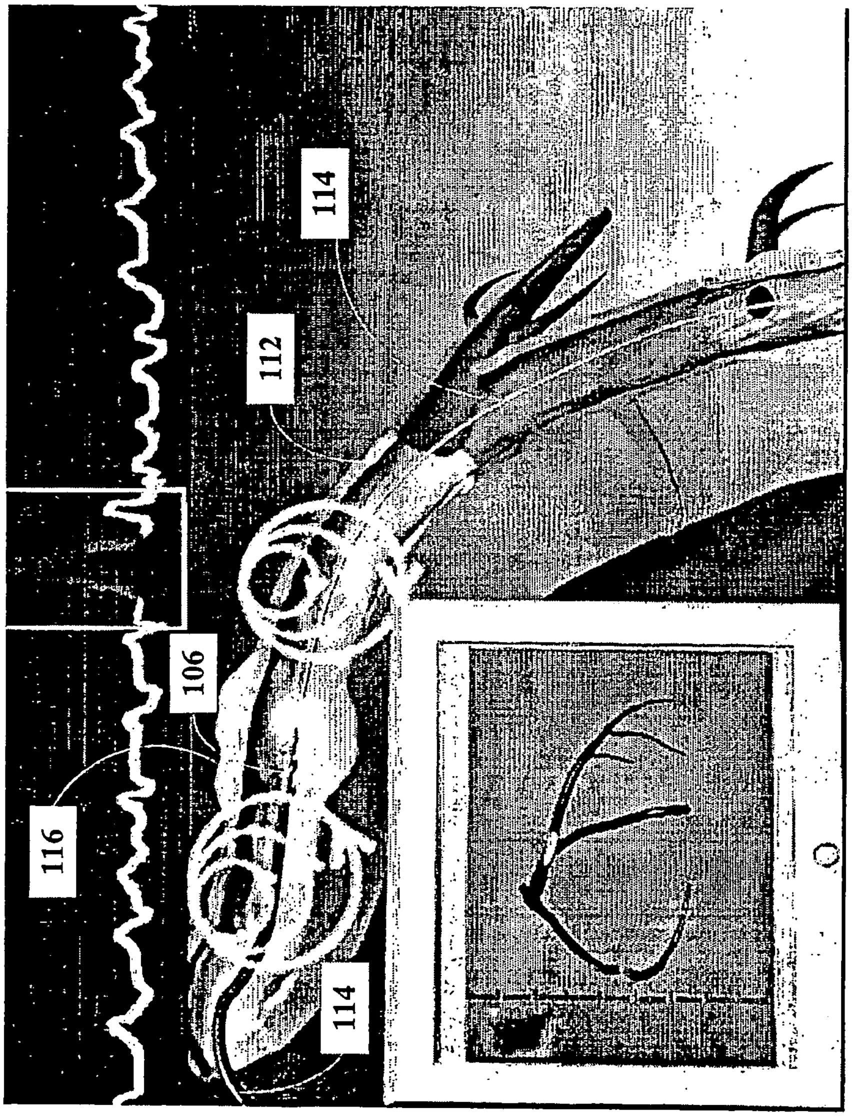


FIG. 1D

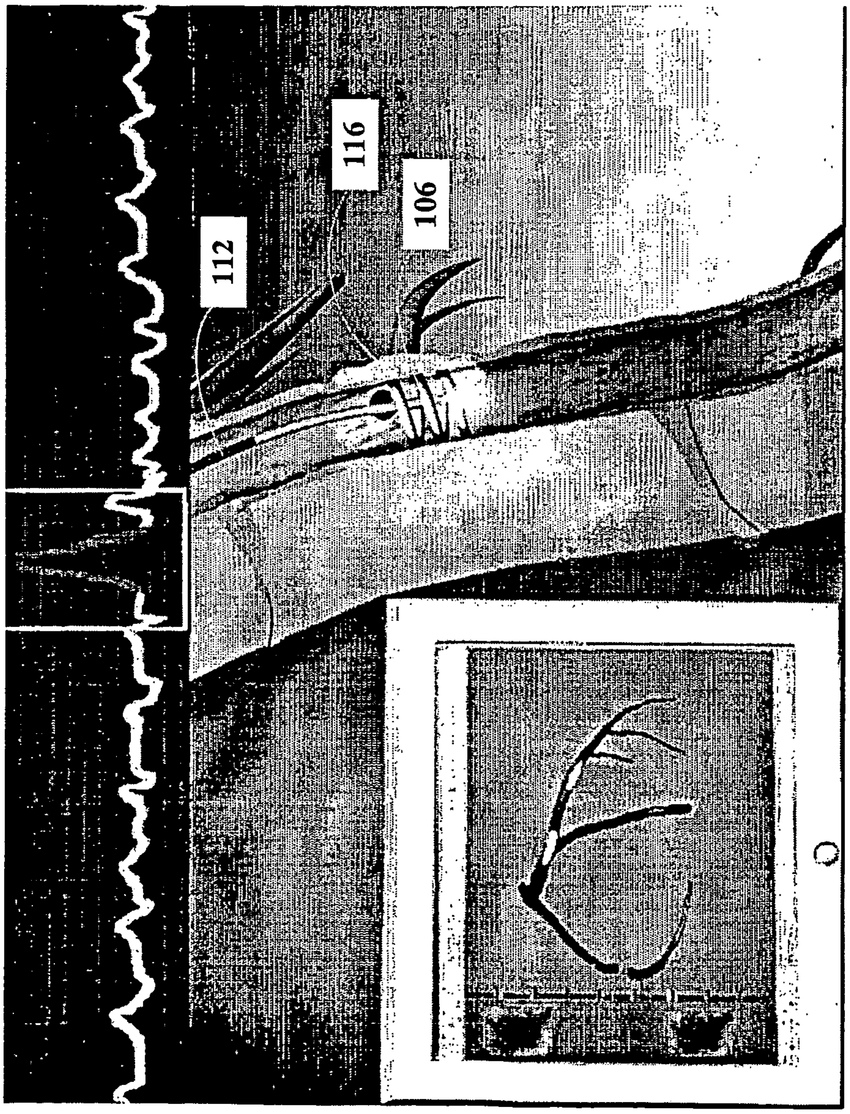


FIG. 1E

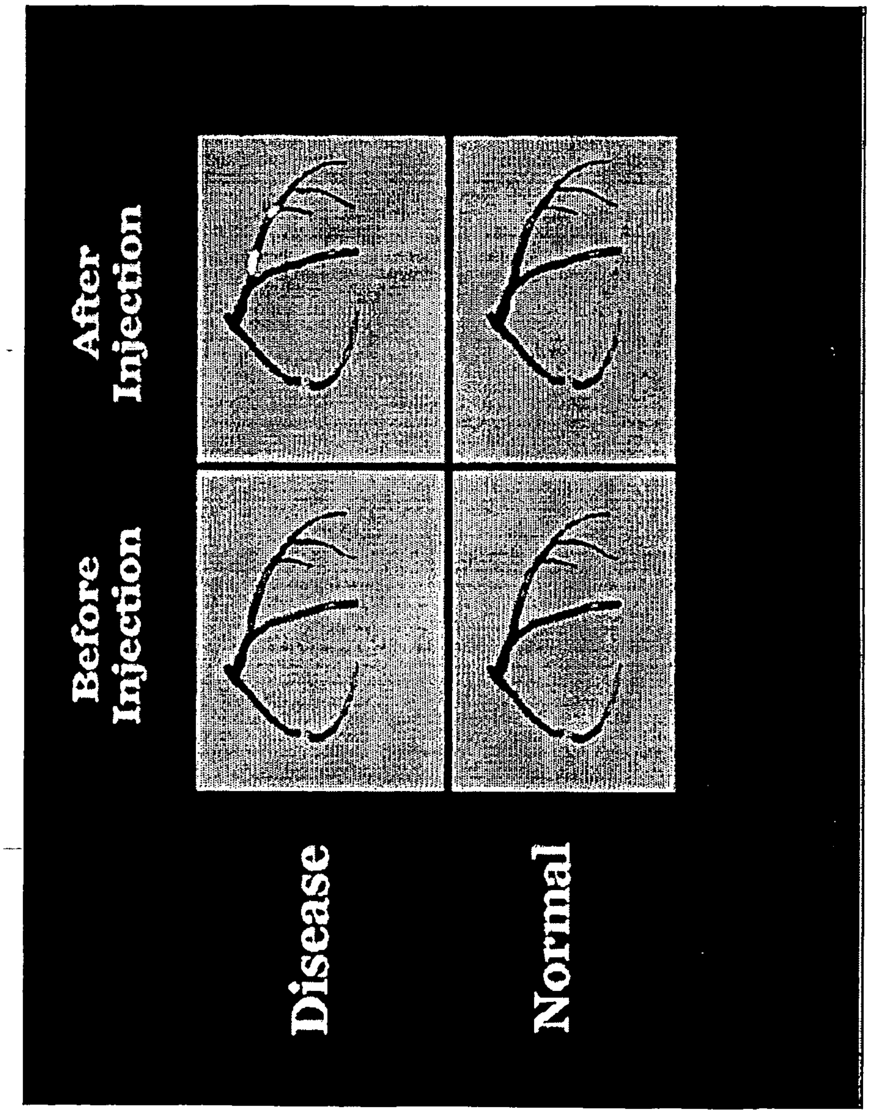


FIG. 1F

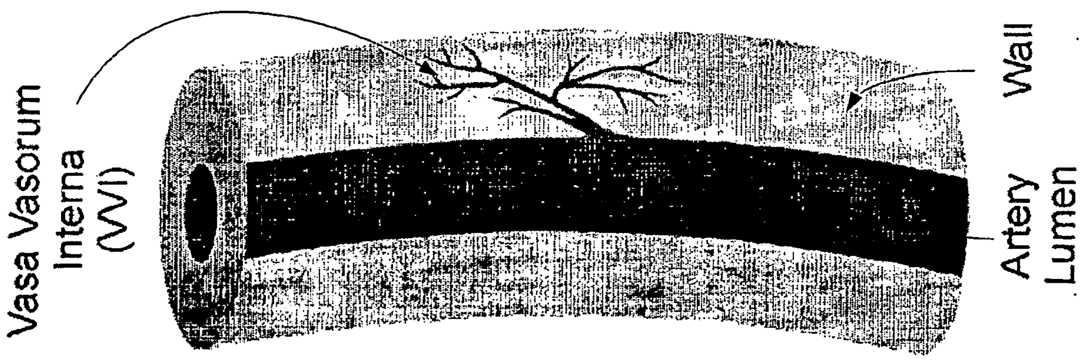


FIG. 2A

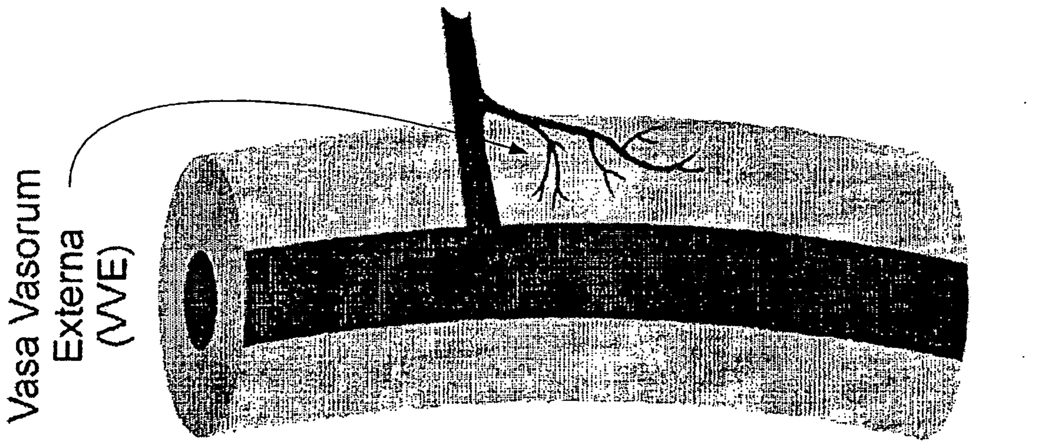


FIG. 2B

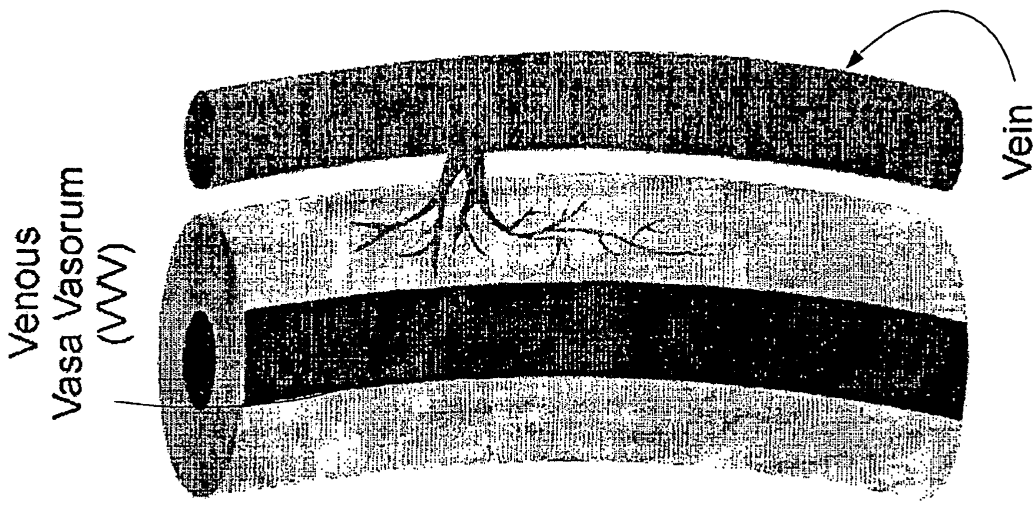


FIG. 2C

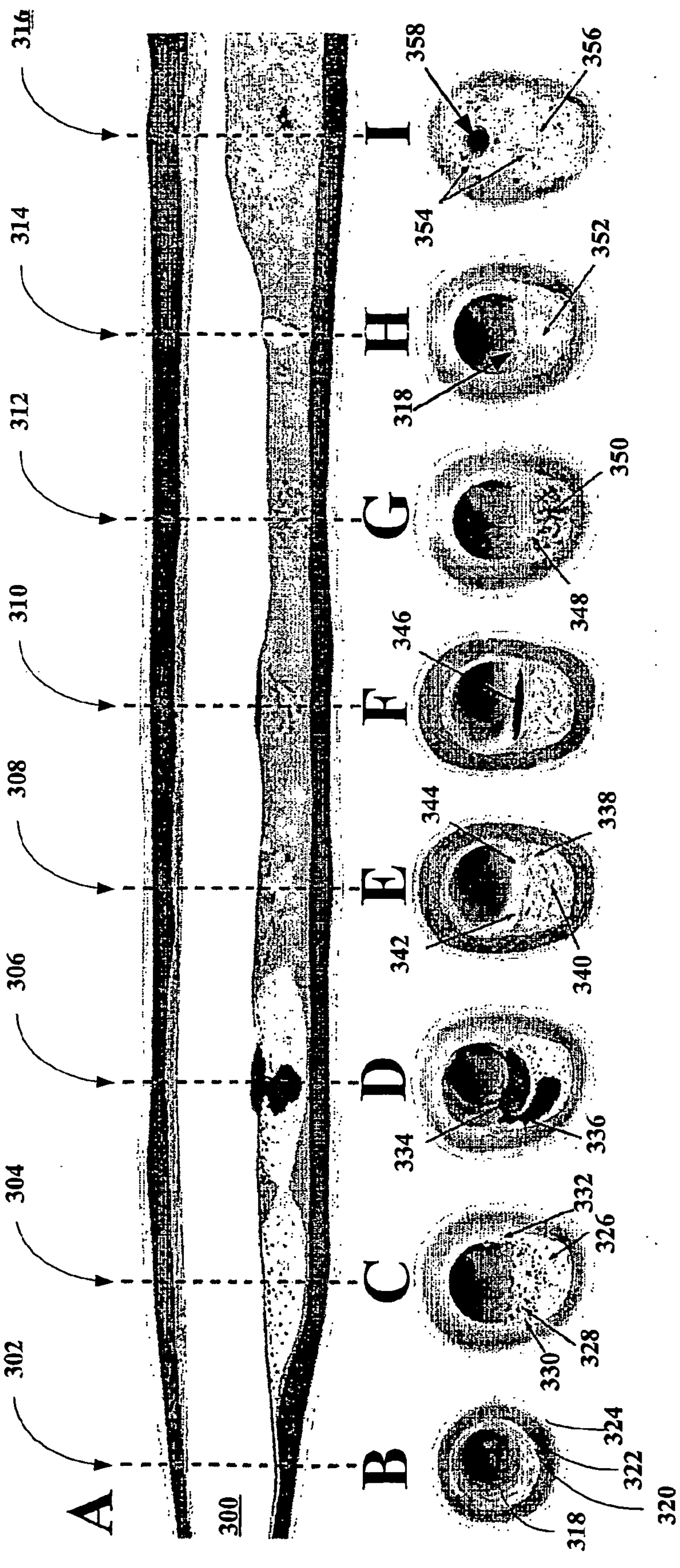
A
300
302
304
306
308
310
312
314
316
B
C
D
E
F
G
H
I
318
320
322
324
326
328
330
332
334
336
338
340
342
344
346
348
350
352
354
356
358

FIGS. 3A-I

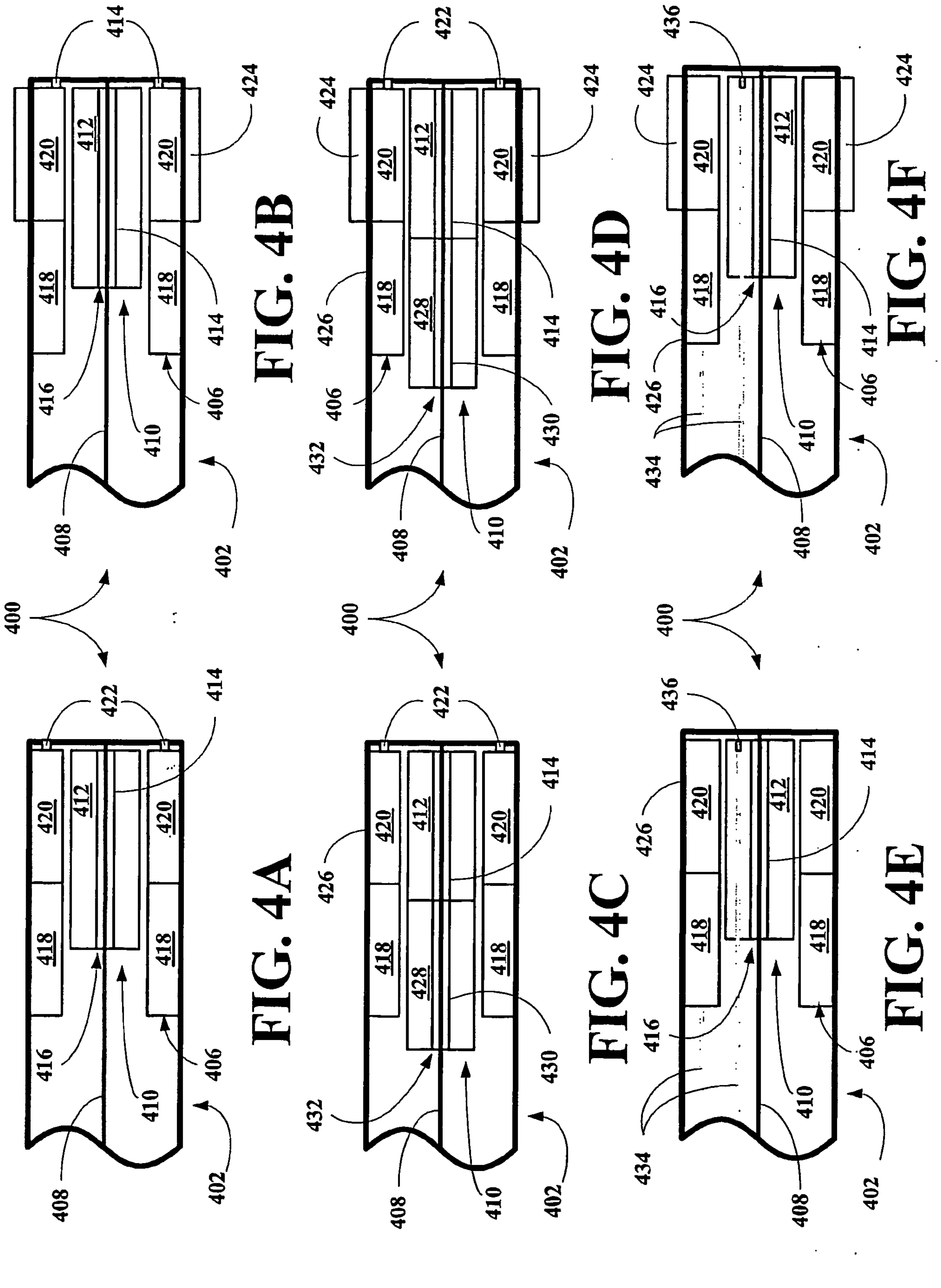


FIG. 4A

FIG. 4B

FIG. 4C

FIG. 4D

FIG. 4E

FIG. 4F

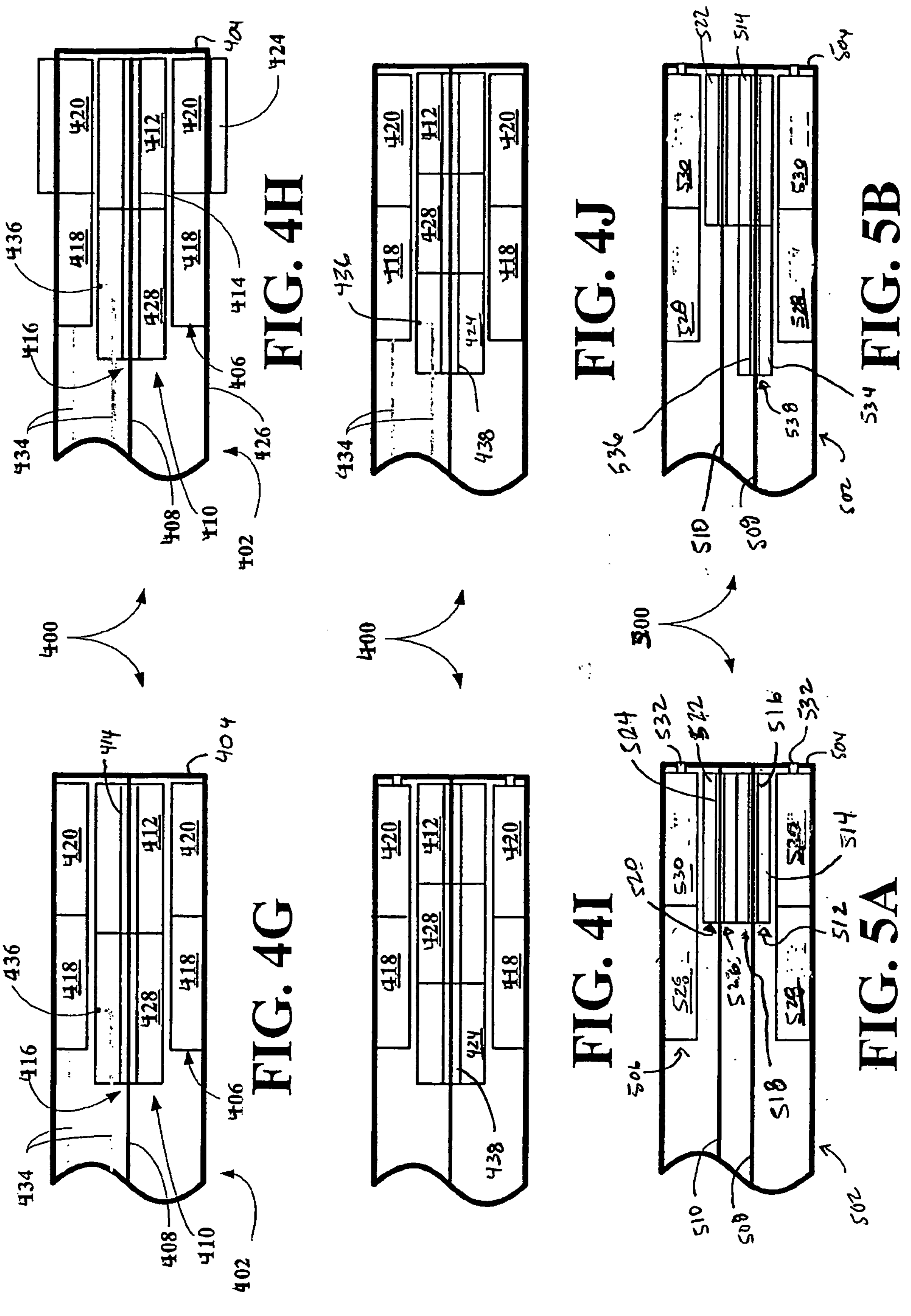

FIG. 4G

FIG. 4H

FIG. 4I

FIG. 4J

FIG. 5A

FIG. 5B

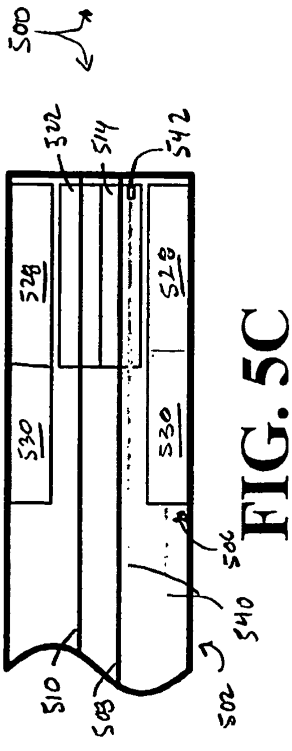
FIG. 5C

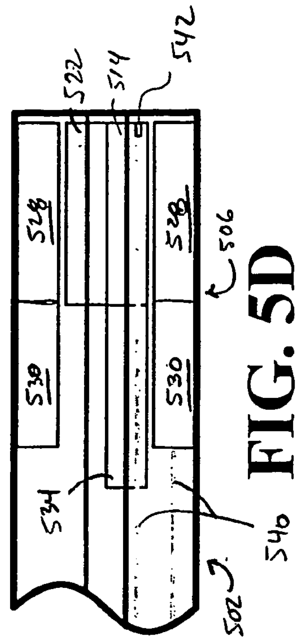
FIG. 5D

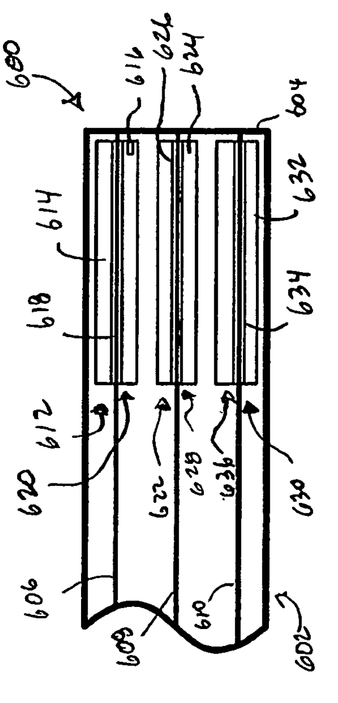
FIG. 6

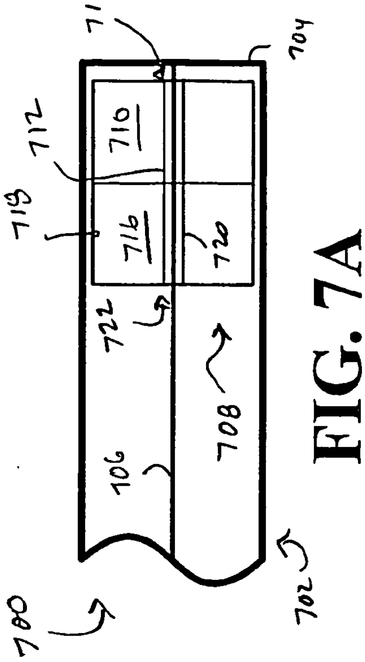
FIG. 7A

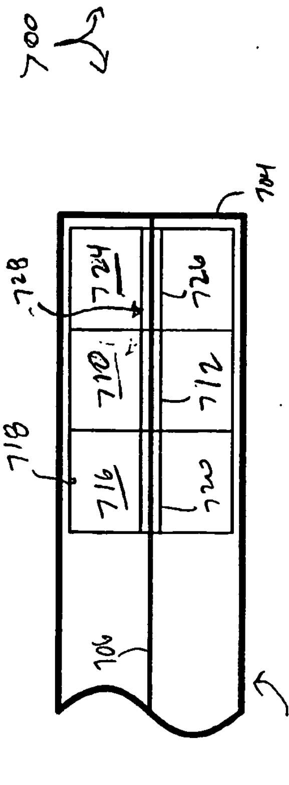
FIG. 7B

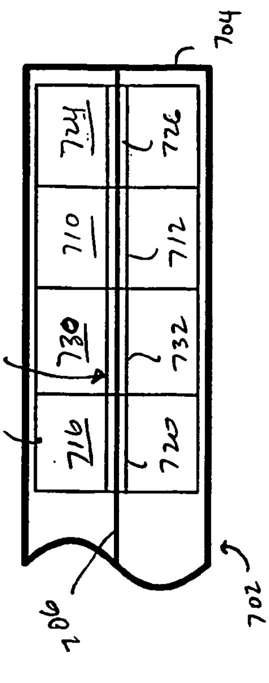
FIG. 7C

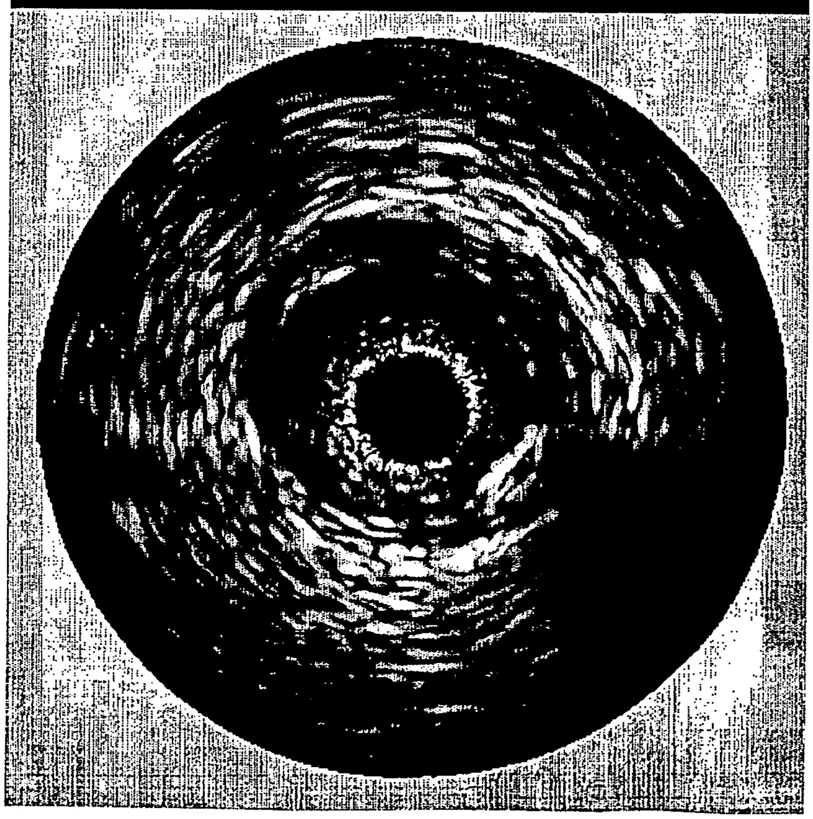

FIG. 8A

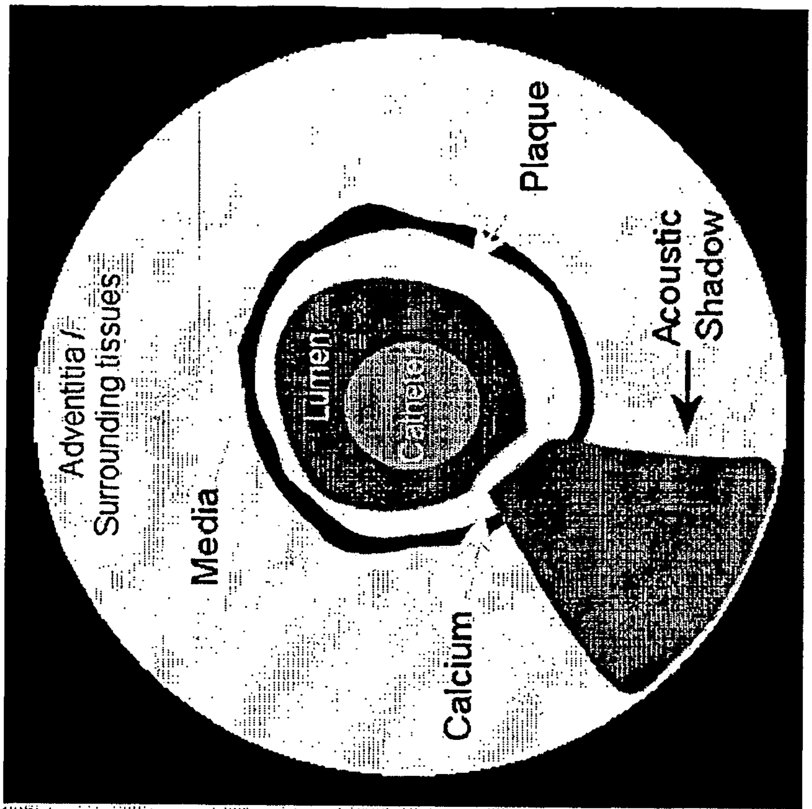
Adventitia /
Surrounding tissues
Media
Lumen
Catheter
Plaque
Calcium
Acoustic
Shadow

FIG. 8B

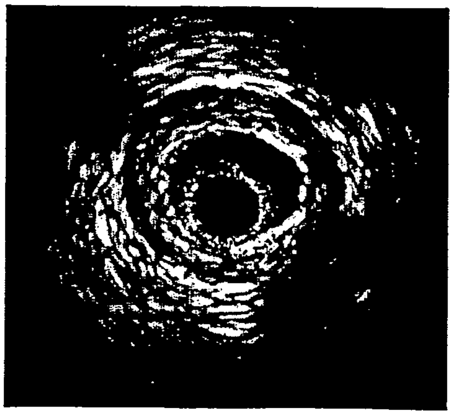
FIG. 9A

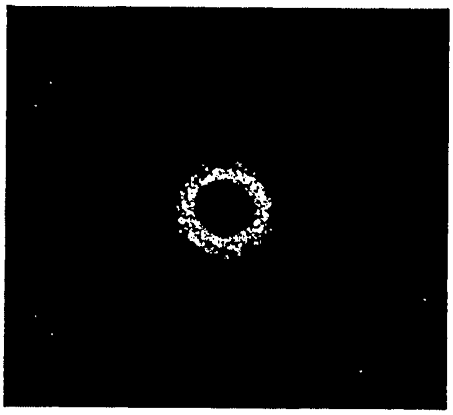
FIG. 9B

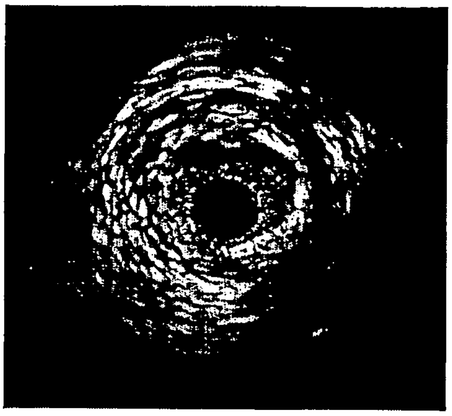
FIG. 9C

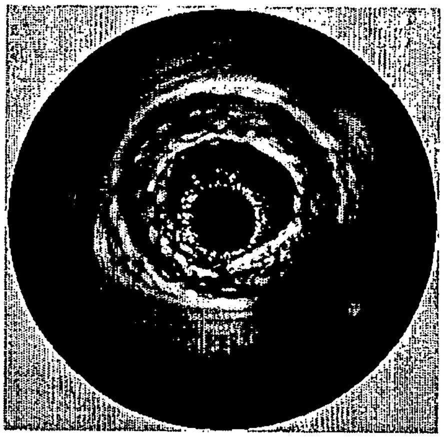
FIG. 10A

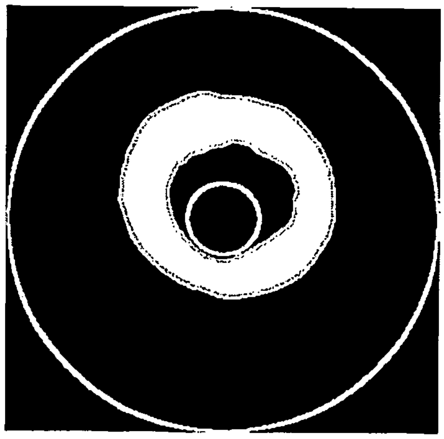
FIG. 10B

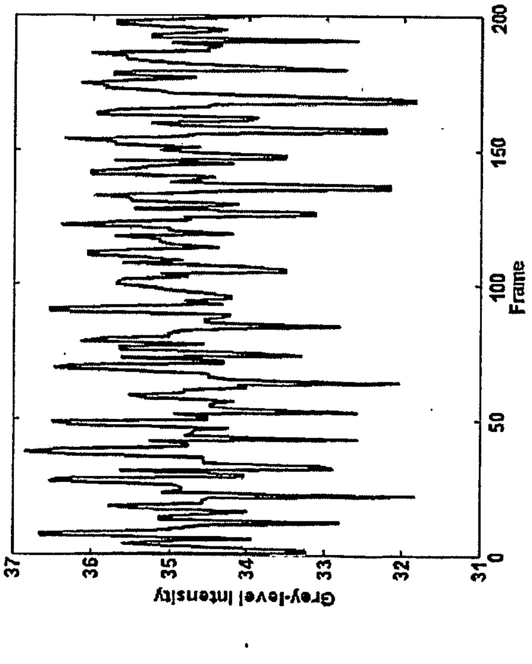


FIG. 11A

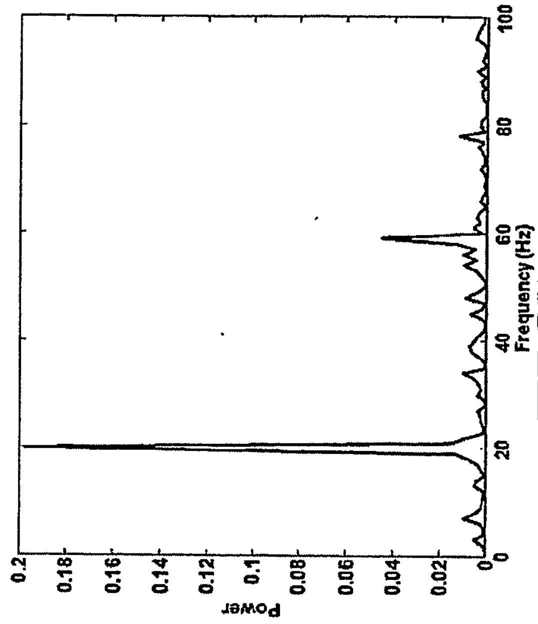


FIG. 11B

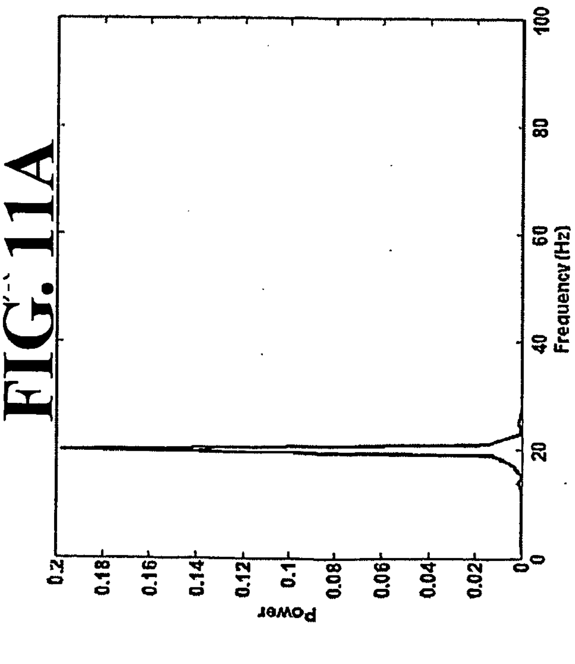


FIG. 11C

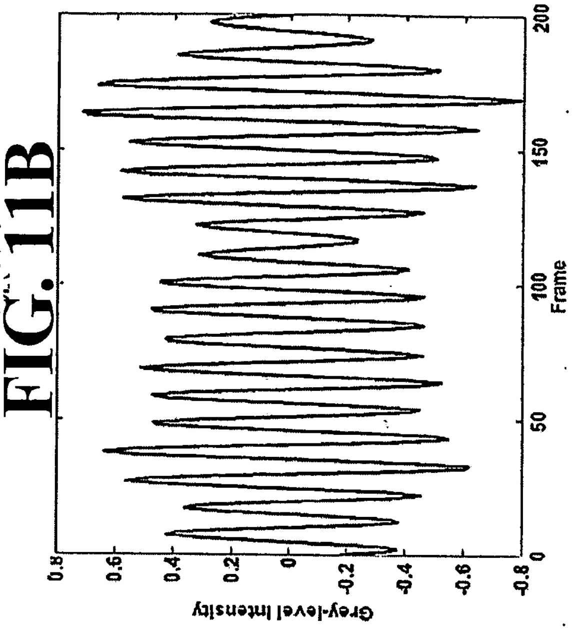


FIG. 11D

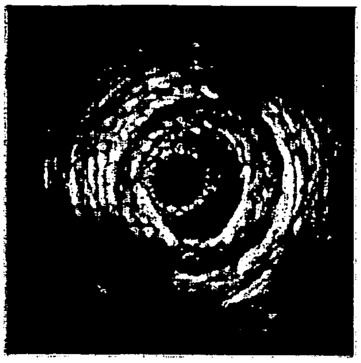
FIG. 12A

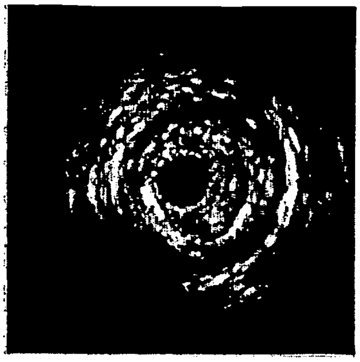
FIG. 12B

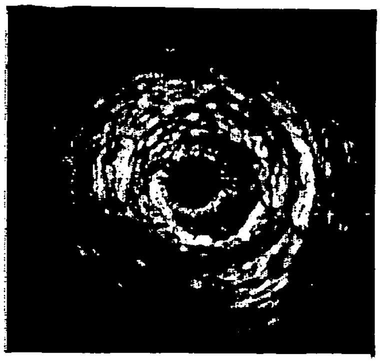
FIG. 12C

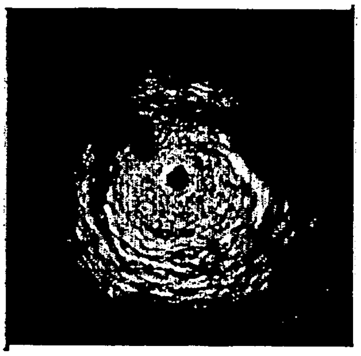
FIG. 12D

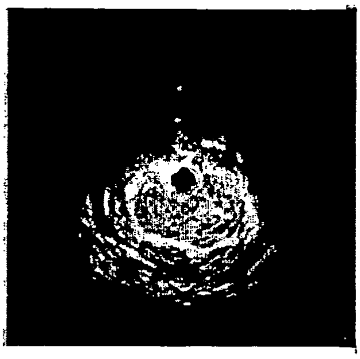
FIG. 12E

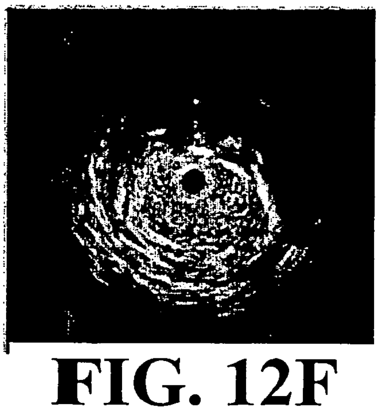
FIG. 12F

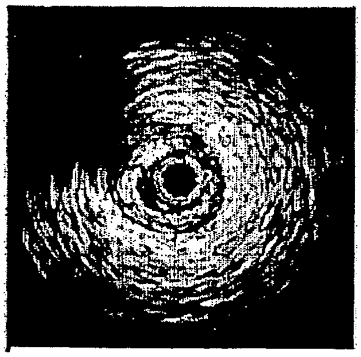
FIG. 12G

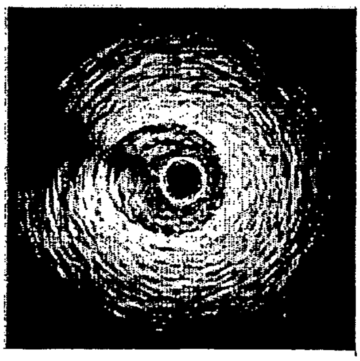
FIG. 12H

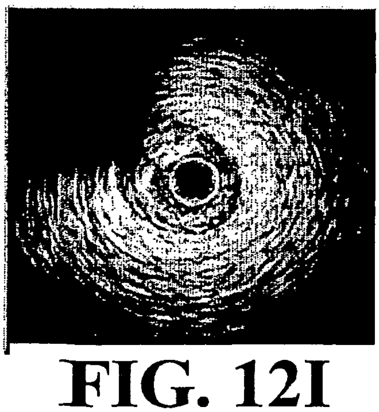
FIG. 12I

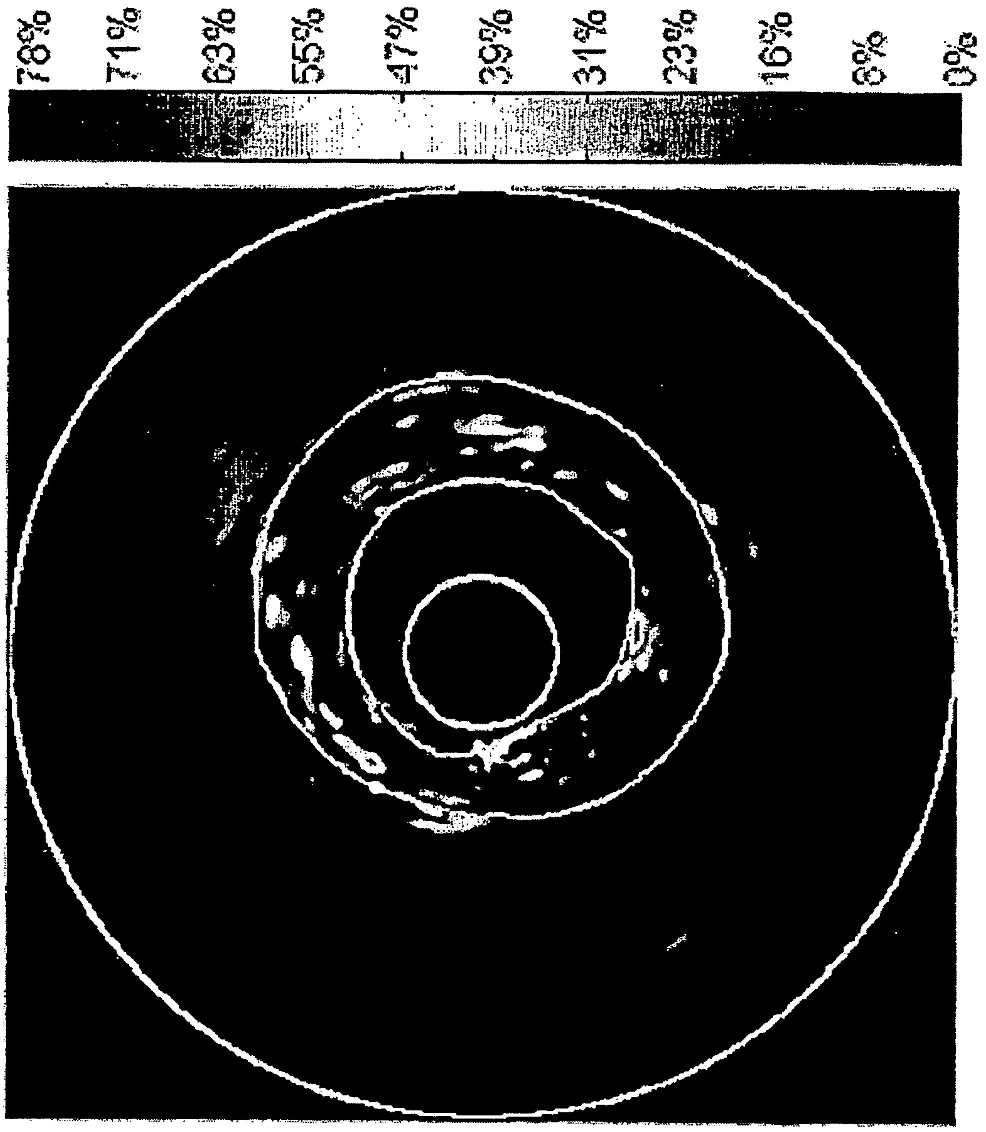


FIG. 13

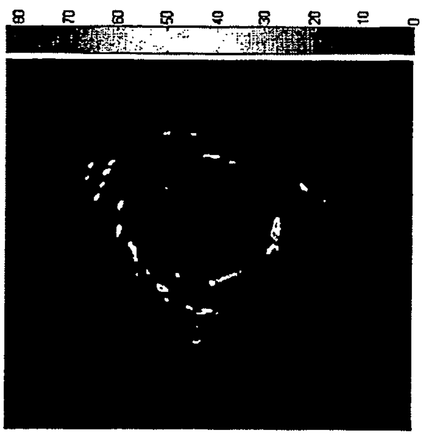

FIG. 14A

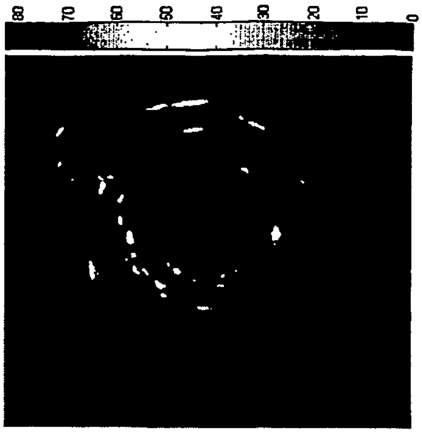

FIG. 14B

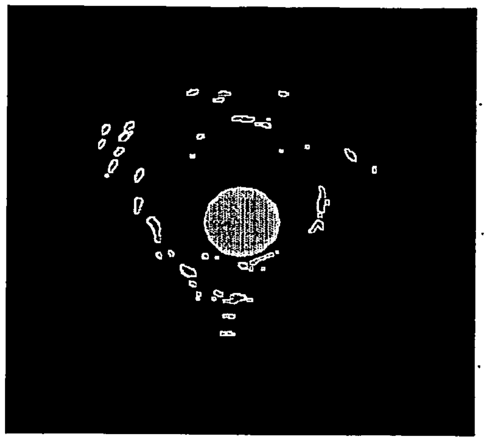
FIG. 15A

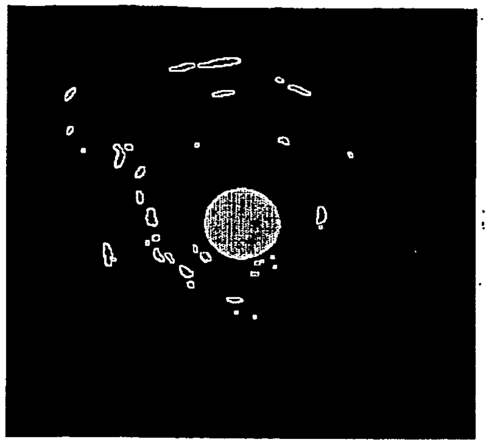
FIG. 15B

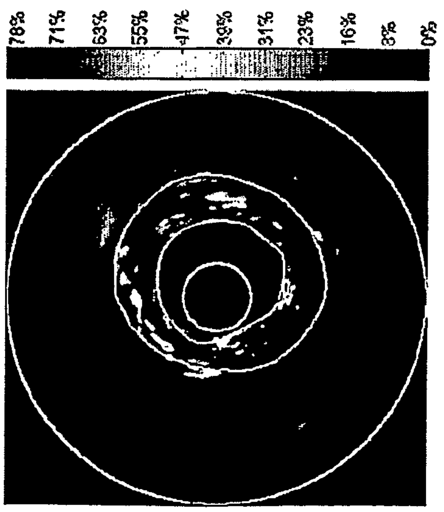

FIG. 3

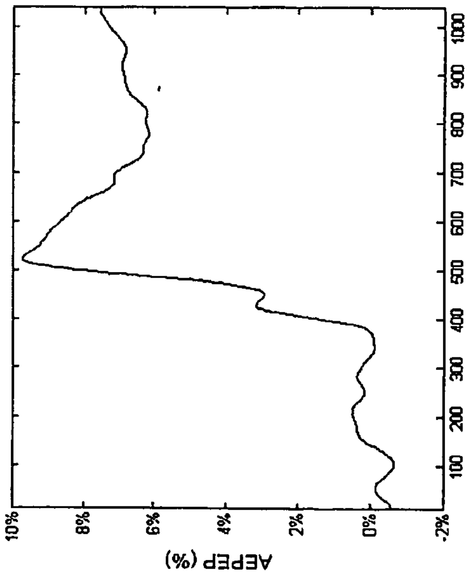


FIG. 16A

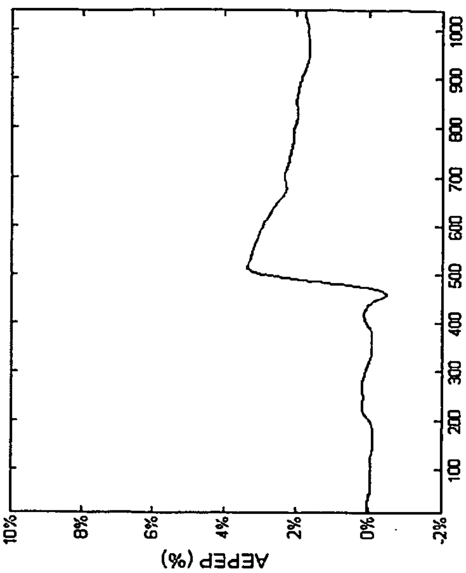


FIG. 16B

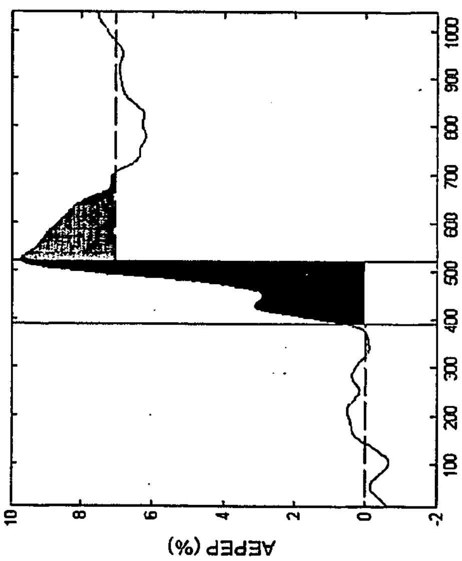


FIG. 17A

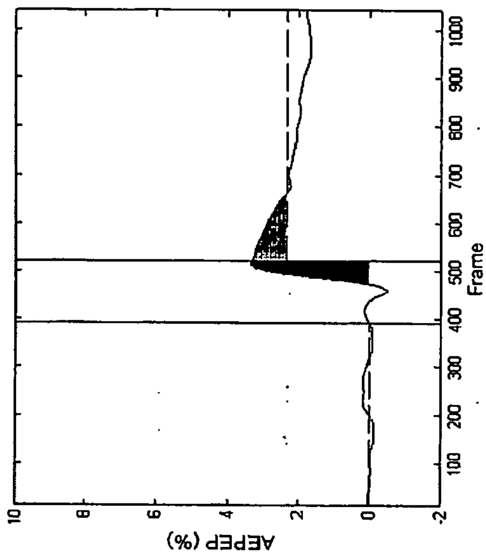


FIG. 17B

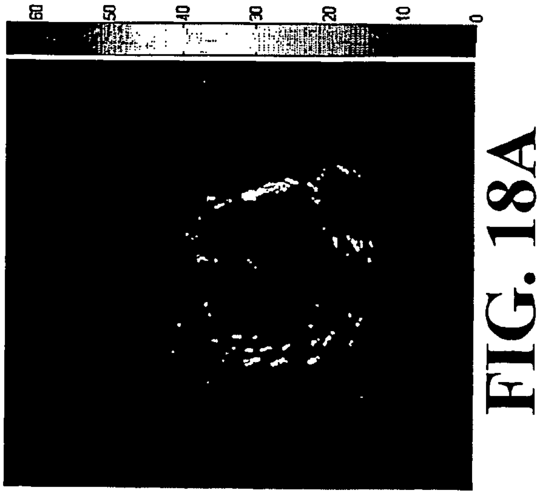


FIG. 18A

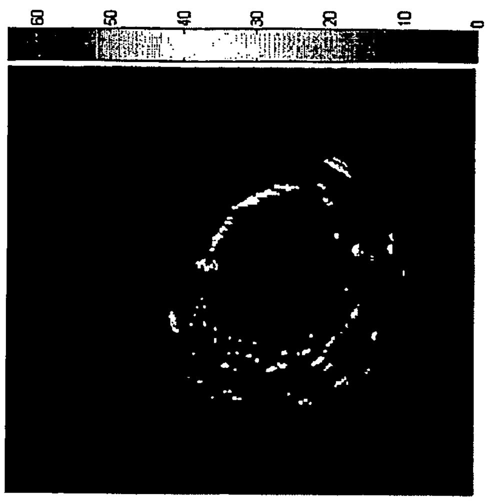


FIG. 18B

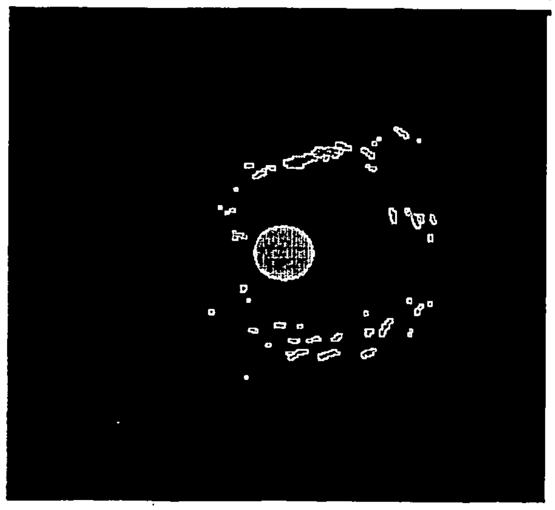

FIG. 19A

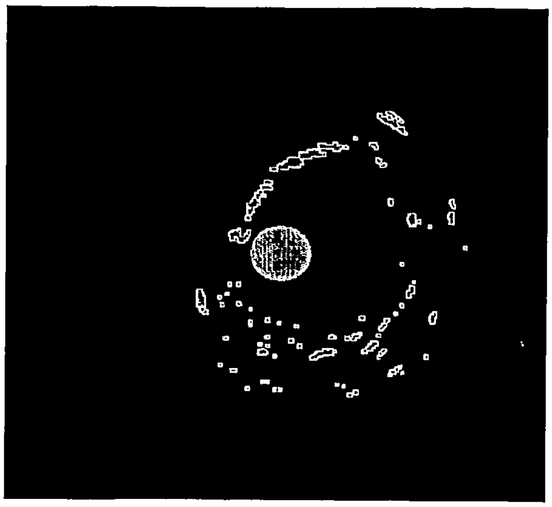

FIG. 19B

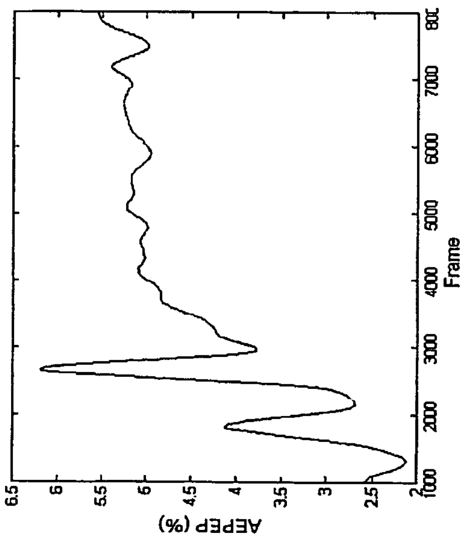


FIG. 20A

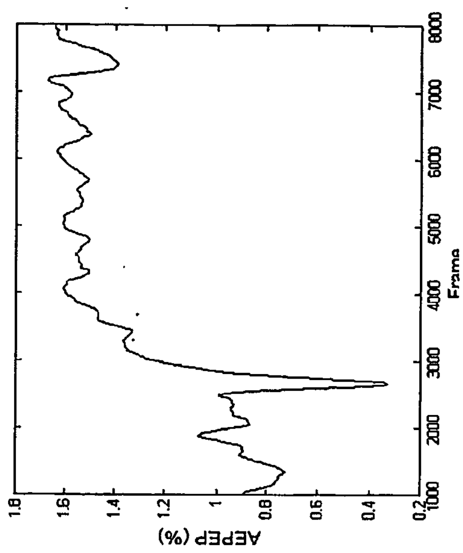


FIG. 20B

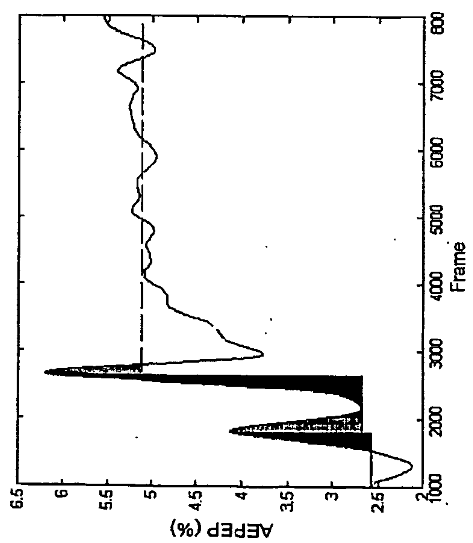


FIG. 21A

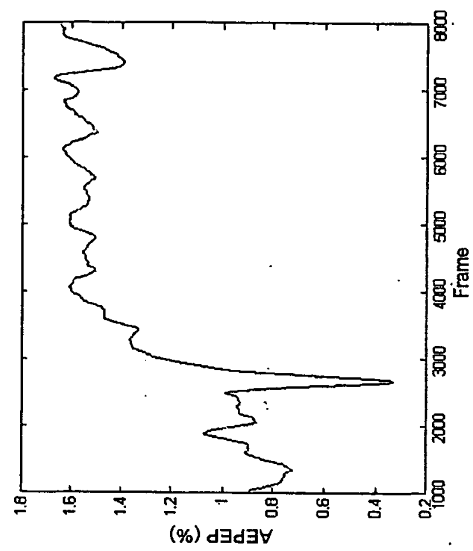


FIG. 21B

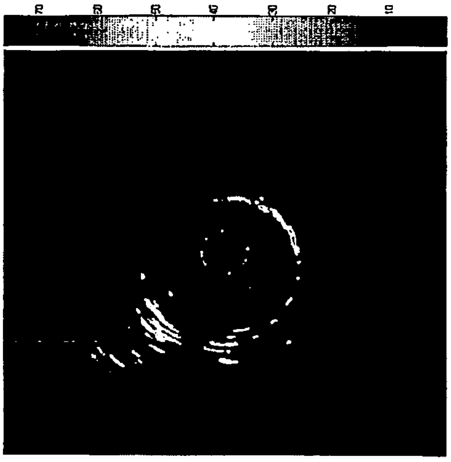

FIG. 22A

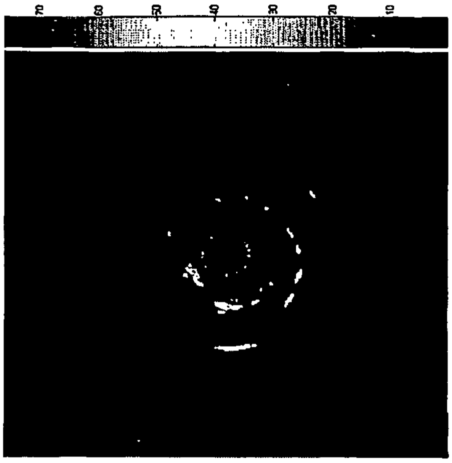

FIG. 22B

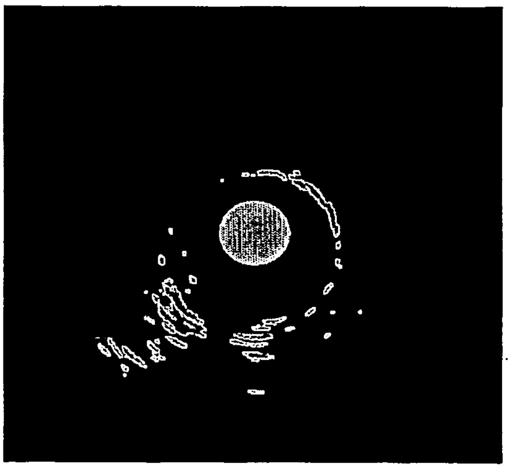

FIG. 23A

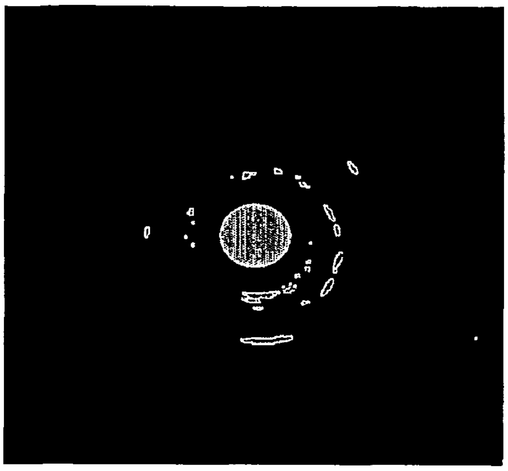

FIG. 23B

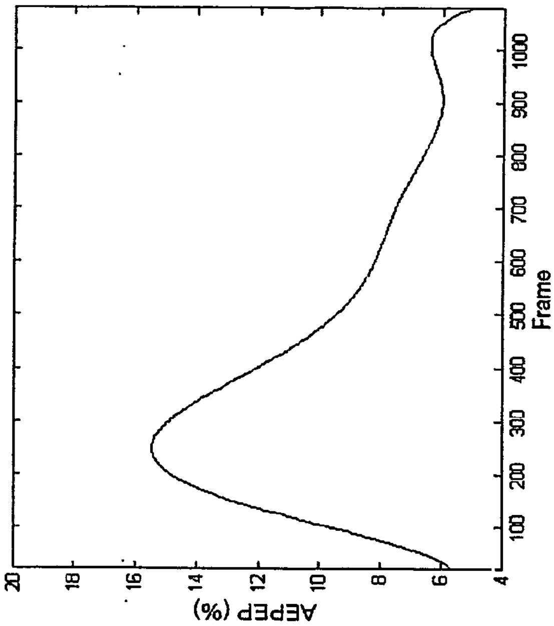


FIG. 24

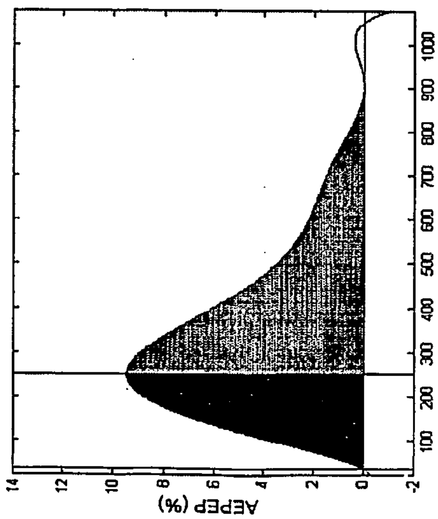


FIG. 25A

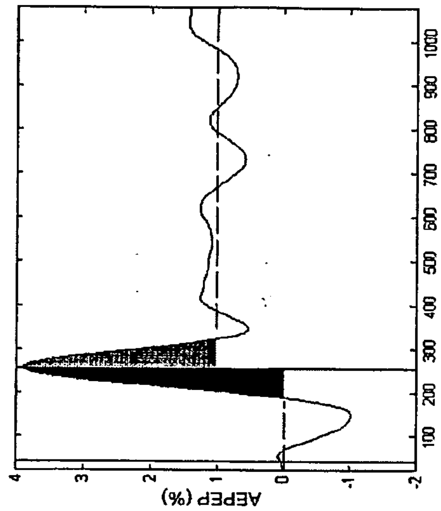


FIG. 25B